# EUROPEAN PATENT SPECIFICATION

(11) **EP 2 069 528 B1**
(45) Date of publication and mention of the grant of the patent: **27.03.2013**
(21) Application number: 08700920.5
(22) Date of filing: 04.02.2008
(51) Int. Cl.: C12Q 1/68, G01N 33/68

(54) **A METHOD FOR DIAGNOSING ATHEROSCLEROTIC PLAQUES BY MEASUREMENT OF CD36**
VERFAHREN ZUR DIAGNOSTIZIERUNG ATHEROSKLEROTISCHER PLAQUE DURCH MESSUNG VON CD36
METHODE DE DIAGNOSTIC DE PLAQUES ATHEROSCLEREUSES PAR MESURE DU RECEPTEUR CD36

(30) Priority: 05.02.2007 DK 200700192
(43) Date of publication of application: 17.06.2009
(73) Proprietor: Region Nordjylland, 9220 Aalborg Øst (DK)
(72) Inventor: HANDBERG, Aase, 8240 Risskov (DK); HALVORSEN, Bente, 0667 Oslo (NO); AUKRUST, Pål, 2322 Ridabu (NO); SKJELLAND, Mona, 0874 Oslo (NO)
(74) Representative: Aagaard, Louise Yung
(86) International application number: PCT/DK2008/000049
(87) International publication number: WO 2008/095492

(56) References cited:
- EP-A- 1 431 399
- WO-A-2004/017986
- WO-A-2005/116644
- WO-A-2005/117859
- JP-A- 2000 180 447
- JP-A- 2001 056 338
- NAKAGAWA-TOYAMA YUMIKO ET AL: "Localization of CD36 and scavenger receptor class A in human coronary arteries: A possible difference in the contribution of both receptors to plaque formation" ATHEROSCLEROSIS, vol. 156, no. 2, June 2001 (2001-06), pages 297-305, XP002487570 ISSN: 0021-9150
- NAKATA ATSUYUKI ET AL: "CD36, a novel receptor for oxidized low-density lipoproteins, is highly expressed on lipid-laden macrophages in human atherosclerotic aorta" ARTERIOSCLEROSIS THROMBOSIS AND VASCULAR BIOLOGY, vol. 19, no. 5, May 1999 (1999-05), pages 1333-1339, XP002487571 ISSN: 1079-5642
- FORCHERON FABIEN ET AL: "Genes of cholesterol metabolism in human atheroma: overexpression of perilipin and genes promoting cholesterol storage and repression of ABCA1 expression." ARTERIOSCLEROSIS, THROMBOSIS, AND VASCULAR BIOLOGY AUG 2005, vol. 25, no. 8, August 2005 (2005-08), pages 1711-1717, XP002487572 ISSN: 1524-4636
- ZINGG J-M ET AL: "Novel 5' exon of scavenger receptor CD36 is expressed in cultured human vascular smooth muscle cells and atherosclerotic plaques" ARTERIOSCLEROSIS, THROMBOSIS, AND VASCULAR BIOLOGY, HIGHWIRE PRESS, PHILADELPHIA, PA, US, vol. 22, 1 March 2002 (2002-03-01), pages 412-417, XP002988565 ISSN: 1524-4636
- FEBBRAIO MARIA ET AL: "Targeted disruption of the class B scavenger receptor CD36 protects against atherosclerotic lesion development in mice" JOURNAL OF CLINICAL INVESTIGATION, vol. 105, no. 8, April 2000 (2000-04), pages 1049-1056, XP002487573 ISSN: 0021-9738
- TSUKAMOTO KAYO ET AL: "Synergically increased expression of CD36, CLA-1 and CD68, but not of SR-A and LOX-1, with the progression to foam cells from macrophages." JOURNAL OF ATHEROSCLEROSIS AND THROMBOSIS 2002, vol. 9, no. 1, 2002, pages 57-64, XP002487574 ISSN: 1340-3478
- NICHOLSON ANDREW C: "Expression of CD36 in macrophages and atherosclerosis: the role of lipid regulation of PPARgamma signaling." TRENDS IN CARDIOVASCULAR MEDICINE JAN 2004, vol. 14, no. 1, January 2004 (2004-01), pages 8-12, XP002487575 ISSN: 1050-1738
- MOORE K J ET AL: "Scavenger receptors in atherosclerosis: Beyond lipid uptake" ARTERIOSCLEROSIS, THROMBOSIS, AND VASCULAR BIOLOGY 200608 US, vol. 26, no. 8, August 2006 (2006-08), pages 1702-1711, XP002487576 ISSN: 1079-5642
- FEBBRAIO M ET AL: "CD36: a class B scavenger receptor involved in angiogenesis, atherosclerosis, inflammation, and lipid metabolism." THE JOURNAL OF CLINICAL INVESTIGATION SEP 2001, vol. 108, no. 6, September 2001 (2001-09), pages 785-791, XP002487577 ISSN: 0021-9738
- ROSSI A ET AL: "Carotid atherosclerotic plaque instability in patients with acute myocardial infarction", INTERNATIONAL JOURNAL OF CARDIOLOGY, ELSEVIER SCIENCE PUBLISHERS, AMSTERDAM, NL, vol. 111, no. 2, 1 December 2005 (2005-12-01), pages 263-266, XP025035635, ISSN: 0167-5273 [retrieved on 2006-08-10]
- KOENIG WOLFGANG ET AL: "Biomarkers of atherosclerotic plaque instability and rupture", ARTERIOSCLEROSIS THROMBOSIS AND VASCULAR BIOLOGY, vol. 27, no. 1, 2 November 2006 (2006-11-02), pages 15-26, ISSN: 1079-5642

## Description

### Technical field of the invention

The present invention relates to diagnosis, classification and monitoring of atherosclerotic plaques using measurement of the concentration of CD36 in a body fluid and/or the plaque as such.

### Background of the invention

Atherosclerosis is a progressive inflammatory disease in which lipids, extracellular matrix, macrophages and activated vascular smooth muscle cells accumulate in the arterial wall resulting in growth of an atherosclerotic plaque. Progression of the atherosclerotic lesions can elicit various acute ischemic events such as acute coronary syndromes, transient ischemic attacks (TIA), and stroke due to parts of the plaque being detached and carried with the blood stream until the plaque stops because the size of the plaque exceeds the size of the artery. However, although the participation of inflammatory mediators in the atherosclerotic process has become widely recognized, the identification of the different components, as well as their relative importance, is unclear. In particular, the way in which inflammation may promote the transition from an asymptomatic fibroatheromatous plaque to a vulnerable and symptomatic lesion is not fully understood.

CD36 has numerous cellular functions. It is a Fatty Acid Translocase (FAT) and belongs to the scavenger receptor Class B family, playing a major role in the uptake of Long Chain Fatty Acids (LCFA) over the cell membrane in metabolically active tissue, in foam cell formation, and in uptake of OxLDL by macrophages. The lipid-rich macrophages are then differentiated into or gain the characteristics of foam cells and contribute to the formation of atherosclerotic lesions. In addition, CD36 of macrophages, together with TSP and the integrin alphavbeta3, can phagocytose apoptotic neutrophils. Furthermore, the protein is one of the receptors of collagen in platelet adhesion and aggregation. Moreover, cytoplasmic CD36 plays an important role in signal transduction by interacting with Src family tyrosine kinases. Deficiency in CD36 in Asian and African populations has been associated with insulin resistance. Alternate names for CD36 (Cluster Determinant 36) include CD36 [HUGO gene name], GPIIIb, GPIV, OKM5-antigen, and PASIV.
EP1431399 relates to a method for identifying therapeutic agents for reducing and monitoring the growth, erosion, rupture or stability of an atherosclerotic plaque comprising the analysis of the differential expression of at least two genes coding proteins chosen among among Stearoyl CoA desaturase, Phosphatidic acid phosphate, and Phosphoinositide-specific-phospholipase-B1.
JP 2000 180447 describes deciding acute myocardial infarction by detecting a CD36 antigen in an organism sample separated from a human and comparing the detected CD36 antigen amount with that of a person with a normal healthy body. Nakagawa-Toyama et al. (Atherosclerosis. 2001 Jun;156(2):297-305) examined the localization of CD36 in human coronary atherosclerotic lesions, and found that the expression of CD36 in these lesions was accelerated in parallel with the progression of atherosclerosis.
Nakata et al. (Arterioscler Thromb Vasc Biol. 1999 May; 19(5): 1333-9) examined the immunohistochemical localization of CD36 in human aorta, and found that CD36 is highly expressed on lipid-laden macrophages in human atherosclerotic aorta.

### Summary of the invention

The present inventors have found that increased concentration of CD36 in a body fluid sample from an individual having an atherosclerotic plaque or in an atherosclerotic plaque as such correlates to an increased risk of said atherosclerotic plaque to become a symptomatic atherosclerotic plaque.

Thus, the present invention relates to methods for diagnosing, classifying and monitoring atherosclerotic plaques, for example in order to distinguish stable atherosclerotic plaques from instable atherosclerotic plaques. In addition it is suggested herein that circulating non-cell bound CD36 protein (soluble CD36, (sCD36)) or a fraction or fragment thereof optionally as part of a lipoprotein complex may be a useful diagnostic or predictive marker for atherosclerotic plaques in blood vessels, in particular in carotid arteries and coronary arteries.

It has been found that the concentration of CD36 both in plasma and in the atherosclerotic plaques increase following the progression of atherosclerotic plaques towards symptomatic atherosclerotic plaques. Accordingly, increase of CD36 concentration may be used as an indication of progression of the atherosclerotic plaques. The method may be used for diagnosing the risk of an individual for acquiring an ischemic event as described herein, and furthermore the method may be used for monitoring an individual for a period observing whether an atherosclerotic plaque is progressing.
Thus, one aspect of the present invention relates to a method for diagnosing an atherosclerotic plaque(s) in an individual, said method comprising in a sample from said individual i) determining the concentration of a CD36 polypeptide, ii) correlating said concentration determined in i) to a standard level, and iii) based on said correlation according to ii) diagnosing said atherosclerotic plaque(s).

In yet a further aspect the present invention pertains to a method for diagnosing stenosis caused by atherosclerotic plaques in an individual, said method comprising in a sample from said individual i) determining the concentration of a CD36 polypeptide, ii) correlating said concentration determined in i) to a standard level, and iii) based on said correlation according to ii) diagnosing the degree of stenosis caused by atherosclerotic plaques in an individual.

In a final aspect the present invention relates to a kit for use in the methods as disclosed herein, wherein the kit comprises at least one detection member. It is appreciated that the detection member is at least one antibody, wherein said antibody is directed against CD36 polypeptide or part thereof. Furthermore, it is to be understood that the detection member is selected from the group consisting of at least one primer, at least one probe and at least one primer pair capable of detecting a CD36 encoding nucleic acid molecule.

### Drawings

Figure 1:
   Soluble CD36 (sCD36) in plasma from patients with carotid atherosclerosis according to the plaque stability. Data are given as function of time period since last clinical symptoms. The carotid stenosis was diagnosed and classified by pre-cerebral color Duplex ultrasound (1) and CT angiography (2) according to consensus criteria. sCD36 is higher in patients who had experienced clinical symptoms within the last 2 months (< 2 mnd), as compared to patients who experienced symptoms 3-6 months (3-6 mnd) or more than 6 months (> 6 mnd), since last symptoms before examination and blood sampling. N = number of patients, p = level of significance ( Mann-Whitney U test). Plasma sCD36 is measured as relative units, i.e. to a pool of EDTA plasma from the routine hospital blood sampling. Clinical symptoms of plaques designate the presence of cerebrovascular symptoms. Symptomatic atherosclerotic plaques are plaques in patients with stroke, TIA (transient ischemic attack), or amaurosis fugax ipsilateral to the stenotic internal carotid artery. Error bars represent SEM.
   (1) Grant EG, Benson CB, Moneta GL et al. Carotid artery stenosis: gray-scale and Doppler US diagnosis - Society of Radiologists in Ultrasound Consensus Conference, Radiology. 2003; 229:340-346
   (2) Anderson GB, Asforth R, Steinke DE et al. CT angiography for the detection and characterization of carotid artery bifurcation disease. Stroke. 2000; 31:2168-2174.
Figure 2:
   Plasma levels of soluble CD36 (sCD36) in patients with atherosclerotic carotid stenosis. The patients were divided into groups according to months (mths) after their last clinical symptoms. Clinical symptoms within the last 2 mths (open bar, n=16), 3- 6 mths (squared bar, n=15), or more than 6 mths (black bar, n=31). *, p< 0.02 versus 2 mths; #, p< 0.02 versus 2 mths.
Figure 3:
   Soluble CD36 (sCD36) in plasma from patients with carotid atherosclerosis. The carotid stenosis was diagnosed and classified by pre-cerebral color Duplex ultrasound (1) and CT angiography (2) according to consensus criteria. The patients were divided into Echolucent (EL) and Echogenic (EG) dependent on ultrasound classification. N = number of patients, p = level of significance (Mann-Whitney U test). Plasma sCD36 is measured as relative units, i.e. to a pool of EDTA plasma from the routine hospital blood sampling. Error bars represent SEM.
   (1) Grant EG, Benson CB, Moneta GL et al. Carotid artery stenosis: gray-scale and Doppler US diagnosis - Society of Radiologists in Ultrasound Consensus Conference, Radiology. 2003; 229:340-346
   (2) Anderson GB, Asforth R, Steinke DE et al. CT angiography for the detection and characterization of carotid artery bifurcation disease. Stroke. 2000; 31:2168-2174.
Figure 4:
   Plasma levels of soluble CD36 (sCD36) in patients with atherosclerotic carotid stenosis. The patients were grouped based on echogenicity after ultrasound examination of the atherosclerotic carotid artery. EL, echolucent plaques (n= 20); EG, echogenic/heterogeneous plaques (n=39). (*), p=0.09.
Figure 5:
   Atherosclerotic plaques from the internal carotid artery from endarterectomy patients were classified into two groups depending on whether or not the patients had experienced ipsilateral stroke, TIA or amaurosis fugax in the prior six months to surgery. Plaques were characterized as symptomatic (n=3) or asymptomatic (n=4) according to the presence or absence of cerebrovascular symptoms, respectively. Soluble CD36 (sCD36) was measured in plasma from blood samples drawn the day prior to surgery. The patients were identical to the patients included in the gene expression profile analysis (microarrays) of carotid plaque (Table 1) with the exception that the blood sample from one of the patients was missing and therefore sCD36 was not measured. N = number of patients. Plasma sCD36 is measured as relative units, i.e. to a pool of EDTA plasma from the routine hospital blood sampling. Error bars represent SEM.
Figure 6:
   Representative photomicrographs of serial sections demonstrating anti-CD36 (A), anti-calprotectin (B), and anti-smooth muscle actin (C) immunostaining in an atherosclerotic carotid lesion removed by endarterectomy from a patient with unstable disease. CD36 immunoreactivity was localized to parts of the lipid-rich core (*) of the lesion with numerous CD68-positive macrophages as seen in panel B. No immunostaining was seen outside the core. Strongest anti-CD36 immunostaining was seen in regions adjacent to the media (m). Panel C demonstrates anti-smooth muscle actin immunostaining in the media. Scale bar 100 µm.

### Detailed description of the invention

### Definitions:

*Atherosclerotic plaques:* the term is used in it's conventional meaning, ie. accumulation in the arterial wall of lipids, extracellular material, inflammatory mediators, inflammatory cells (i.e. leukocytes), macrophages and vascular smooth muscle cells.

*Symptomatic atherosclerotic plaque:* an atherosclerotic plaque in an individual that has suffered from symptoms, for example in the form of ipsilateral stroke, transient ischemic attack, or amaurosis fugax.

*Asymptomatic atherosclerotic plaque:* an atherosclerotic plaque in an individual that has not experienced the symptoms mentioned under symptomatic atherosclerotic plaque within the past six months or more.

*Stable atherosclerotic plaque:* an atherosclerotic plaque in an individual that shows no sign of progression during a predefined time-period of six months or more.

*Instable atherosclerotic plaque:* an atherosclerotic plaque in an individual show signs of progression.

In the present context the term atheromatous plaque is the accumulation and swelling in artery walls where the swelling is caused by for example macrophages, cell debris, lipids such as cholesterol and fatty acids, calcium and fibrous connective tissue. Thus, the process of atheroma development within an individual is called atherogenesis and the overall result of the disease process is termed atherosclerosis. Therefore, the term atheromatous plaque is used interchangeably with atherosclerotic plaque in the present invention.

By the term 'CD36 encoding nucleic acid molecule' is meant a transcriptional product of the gene encoding CD36.

*Diagnosis:* as used herein refers to methods by which the skilled person can estimate and/or determine whether or not a patient is suffering from a given disease or condition. The skilled person often makes a diagnosis on the basis of one or more diagnostic indicators, i.e., in the amount or concentration of a marker, or change in amount of marker which is indicative of the presence, severity, or absence of the condition.

The term *correlating* as used herein, refers to comparing the presence or amount of the marker(s) in a patient to its presence or amount in individuals suffering from or at risk of suffering from, a given condition; or in persons known to be free of a given condition. As discussed above, a marker level in a patient sample can be compared to a level known to be associated with a specific diagnosis. The sample's marker level is said to have been correlated with a diagnosis; that is, the skilled person can use the marker level to determine whether the patient suffers from a specific type diagnosis, and respond accordingly. Alternatively, the sample's marker level can be compared to a marker level known to be associated with a good outcome (e.g., the absence of disease, etc.).
It is appreciated that the term 'marker' in the present invention refers to CD36 polypeptide and/or CD36 encoding nucleic acid molecule.

*Determining the diagnosis* as used herein refers to methods by which the skilled person can determine the presence or absence of a particular disease in a patient. The term "diagnosis" does not refer to the ability to determine the presence or absence of a particular disease with 100% accuracy. Instead, the skilled person will understand that the term "diagnosis" refers to an increased probability that a certain disease is present in the subject. In preferred embodiments, a diagnosis indicates about a 5% increased chance that a disease is present, about a 10% chance, about a 15% chance, about a 20% chance, about a 25% chance, about a 30% chance, about a 40% chance, about a 50% chance, about a 60% chance, about a 75% chance, about a 90% chance, and about a 95% chance. The term "about" in this context refers to +/- 2%.

### Diagnosis, classification and monitoring atherosclerotic plaque status

The present invention is based on the analysis of plaques and blood samples from patients who has suffered or suffers from internal carotid stenoses and were treated by carotid endarterectomy or carotid angioplasty with stenting. Based on the outcome of a study of the concentration of CD36 polypeptide in samples from patients the present invention allows for monitoring progression toward an symptomatic plaque.

The destabilization of an atherosclerotic plaque leading to the progression of an atherosclerotic plaque from asymptomatic to symptomatic is important to diagnose, classify, and monitor since the destabilization can elicit various acute ischemic events, such as stroke, transient ischemic attack, and acute coronary syndromes.

The present invention relates to a method for diagnosing atherosclerotic plaques in an individual in order to predict and prevent acute ischemic events due to destabilization of the plaques.

The method may be used for diagnosing, classifying and monitoring atherosclerotic plaques in any artery, in particular in the carotid arteries and the coronary arteries as well as a method for determining whether an individual is at risk of having and/or acquiring a symptomatic atherosclerotic plaque.

Also, the present invention relates to a method for determining the degree of stenosis caused by an atherosclerotic plaque, by measuring the concentration of CD36 polypeptide or CD36 nucleic acid molecule.

Thus, the present invention relates in one aspect a method for diagnosing atherosclerotic plaques in an individual, said method comprising in a sample from said individual
i) determining the concentration of a soluble CD36 polypeptide, ii) correlating said concentration determined in i) to a standard level, and iii) based on said correlation according to ii) diagnosing said atherosclerotic plaques.

The classification and/or diagnosis of atherosclerotic plaques involves resolving whether an asymptomatic plaques is destabilized and has a higher risk of progressing into symptomatic plaques which may result in the development of acute ischemic events. In the present invention a correlation has been found between the concentration of CD36 polypeptide and the destabilization of asymptomatic plaques to symptomatic plaques. Thus, an increase in the concentration of CD36 polypeptide compared to a standard level is indicative of destabilization of asymptomatic plaques progressing into symptomatic plaques.

It is within the scope of the present invention to monitor the progression of destabilization of atherosclerotic plaques, whether asymptomatic or symptomatic in an individual. The concentration of CD36 polypeptide is determined as described in step i) and correlated to a standard level. The outcome of the correlation can be compared to concentrations of CD36 polypeptide determined at other time points in said individual. By monitoring the concentration of CD36 polypeptide in an individual at different time points the progress of the destabilization of atherosclerotic plaques can be followed. Such monitoring of an individual can be useful for determining the treatment regime of an individual.

Furthermore, the present invention relates to a method for diagnosing stenosis caused by atherosclerotic plaques in an individual, said method comprising in a sample from said individual i) determining the concentration of a CD36 polypeptide, ii) correlating said concentration determined in i) to a standard level, and iii) based on said correlation according to ii) diagnosing the degree of stenosis caused by atherosclerotic plaques in an individual.

The present invention relates to atherosclerotic plaques in all types of arteries of the body, for example carotids, coronary arteries, renal artery or arteries supplying the arms and legs. Below are listed some of the symptoms that are associated with atherosclerosis and the destabilization of atherosclerotic plaques and an increased risk of progression into symptomatic atherosclerotic plaques.

A stenosis is an abnormal narrowing in a blood vessel. Stenosis of the vascular type are often associated with a noise (bruit) resulting from turbulent flow over the narrowed blood vessel. This bruit can be made audible by a stethoscope. However, other more precise methods of diagnosis includes ultrasound, magnetic resonance imaging/magnetic resonance angiography, computed tomography/CT-Angiography which display anatomic imaging and/or flow phenomena.

Within the scope of the present invention are also methods related to determination, classification, diagnosing, monitoring coronary heart disease (CHD), which is also known as coronary artery disease (CAD), ischemic heart disease, atherosclerotic heart disease. Coronary heart disease is the end result of the accumulation of atheromatous plaques within the walls of the arteries that supply the myocardium with oxygen and nutrients.

Typically, the symptoms of coronary heart disease are noted only in the advanced state of disease. The majority of individuals suffering from coronary heart disease do not show any evidence of disease for years and even decades while the disease progresses until the first onset of symptoms, often a "sudden" heart attack, arises. The reason for this disease progression is often rupture of atheromatous plaques may rupture and start limiting blood flow to the heart muscle.
Acute myocardial infarction which is also commonly known as a heart attack is also a condition to which the present invention relates. Acute myocardial infarction is a condition which involves the interruption of the blood supply to part of the heart. The interruption of the blood supply is often brought on by the rupture of an atheroclerotic plaque of the inner arterial wall. The atherosclerotic plaque may block the passage of blood in the artery which results in ischemia or oxygen shortage which causes damage and potential death of heart tissue.
Individuals at risk of having and/or acquiring an acute myocardial infarction are normally characterized by the use of risk factors, such as a previous history of vascular disease for example atherosclerotic coronary heart disease and/or angina, a previous heart attack or stroke.

Angina pectoris is mainly caused by coronary artery disease is chest pain due to lack of blood and hence oxygen supply of the heart muscle which is generally due to obstruction or spasm of the coronary arteries. Thus, according to one embodiment of the invention the methods and kits herein are also related to determine whether an individual is at risk of having/acquiring angina pectoris caused by atherosclerotic plaque.

Carotid stenosis is a narrowing of the lumen of the carotid artery, usually caused by atherosclerosis. The unqualified term "carotid stenosis" in common medical usage refers to the stenosis in the proximal part of the internal carotid artery (at the carotid bulb), as this is the by far the most common site of stenosis within the carotid arteries. Stenosis in other parts of the carotid arteries does occur. Thus, in the present context the term 'carotid stenosis' covers the narrowing in at least one of the carotid arteries, and in particular the proximal part of the internal carotid artery.
Atherosclerotic carotid stenosis may be asymptomatic or it may cause symptoms by embolism to either cerebral vessels in the brain or to the retinal arteries. The term emboli as used herein is used in its conventional meaning, namely the event in which an embolus (object) through circulation migrates from one part of the body and causes a blockage of a blood vessel in another part of the body. Emboli to the cerebral arteries cause transient ischaemic attack (TIA) or cerebrovascular accident (CVA). Emboli to the retina produce amaurosis fugax or retinal infarction.

The diagnosis of carotid stenosis is often determined by colour flow duplex ultrasound scan of the neck arteries. This test has moderate sensitivity and specificity and yields many false-positive results.

The methods and kits of the present invention also relate to a transient ischemic attack, TIA, which is often referred to as a "mini stroke". TIA is caused by the temporary disturbance of blood supply to a restricted area of the brain. The disturbance of the blood supply to the brain often results in brief neurologic dysfunction that usually persists for less than 24 hours. The symptoms associated with TIA vary widely depending on the area of the brain affected by the TIA. Frequently encountered symptoms include temporary loss of vision (typically *amaurosis fugax);* difficulty speaking (aphasia); weakness on one side of the body (hemiparesis); and numbness or tingling (paresthesia), usually on one side of the body. Impairment of consciousness is very uncommon. If there are neurological symptoms persisting for more than 24 hours, it is classified as a cerebrovascular accident, or stroke.

The clinical feature of stroke or cerebrovascular accident (CVA) is a rapidly developing loss of brain function due to a disturbance in the blood supply to the brain. This disturbance may be due to lack of blood supply (ischemia) caused by thrombosis or embolism, or due to a hemorrhage. The traditional definition of stroke is of a "neurological deficit of cerebrovascular cause that persists beyond 24 hours or is interrupted by death within 24 hours". In the present invention the methods and kits are related to stroke caused by atherosclerotic plaques which cause disturbance in the blood supply to the brain.
Previous stroke or transient ischemic attack are typical risk factors of stroke.

### CD36

For the purposes of the present invention the term "CD36" used in phrases, such as "circulating CD36", or "non-cell bound CD36", and in the claims, includes CD36 protein or a fragment thereof which is recognised by a specific antibody against CD36, such as sc5522, sc9154, and sc7309. This includes full length CD36 protein, a polypeptide or peptide fragment thereof, as well as such protein or fragment(s) thereof present in a high molecular weight plasma fraction complex comprising lipoprotein thereof all of which are preferably soluble (sCD36). The terms circulating CD36 and soluble CD36 (sCD36) may be used as synonyms herein. The circulating CD36 may be secreted from caveolae or which is part of a microparticle comprising membrane portions originating from a caveola membrane which has been released from a cell membrane and is present in the blood circulation. Caveolae of interest in this connection may be present in the cell membrane of cells, such as adipocytes, macrophages, monocytes and thrombocytes.

CD36 is a 471 amino acid, transmembrane protein (having 1 or 2 membrane spanning domains at amino acid residue positions 439-460 and possibly 7-28). CD36 is a highly glycosylated, 88kDa glycoprotein with palmitoyl binding sites. CD36 is present in caveolae where it may play a role in the mediation of cellular cholesterol movement into and out of cells.

### Molecular families in which CD36 is a member:

CD36-->SR-B class->host defence scavenger receptors-->scavenger receptor super-family

### Molecular structure of CD36 :

471 amino acid residues;
Transmembrane region (residues 439-465) and,
438 amino acid amino-terminal region may be entirely extracellular or may have a second potential transmembrane region near the amino terminal end;
aa short cytoplasmic tail (residues 466-471);
Within the extracellular region resides a hydrophobic region which probably associ-ates with the outer cell membrane (residues 184-204).

The molecular mass of CD36 is reported to be dependent on cell type as shown below:
Platelets 88 kDa / 113 kDa
Fetal Erythrocytes 78 kDa
Mammary epithelial Cells 85 kDa
Erythroleukeimic 88 kDa, 85 kDa, 57 kDa8
HeLa 85 (160) kDa 85 kDa
Dermal Microvascular endothelial cells 80-90 kDa
In post-transcriptional modification of CD36 two alternate CD36 mRNA forms have been identified. The first mRNA type is expressed in HEL cells and omits amino acid residues 41-143. The second mRNA type has not yet been translated but in which the last 89 residues have been omitted. Thus, CD36 is also found in forms in which the open reading frame is 90-1708 and 357-1775, respectively.

### Post-translational modification of CD36:

CD36 is purported to be heavily glycosylated, with 10 N-linked glycosylation sites in the extracellular portion. Glycosylation has been suggested to confer its resistance to proteolytic cleavage;
Threonine 92 has been shown to be phosphorylated;
CD36 is also palmitoylated on both N- and C-terminal cytoplasmic tails.

Proteins and DNA elements which regulate transcription of CD36 molecule:
Oct-2: The first gene shown to be regulated by the Oct-2 transcription factor during B cell differentiation;
PEBP2: The PEBP2/CBF transcription factors may be important for the constitutive expression of CD36 in monocyte;
CBF: The PEBP2/CBF transcription factors may be important for the constitutive expression of CD36 in monocyte.

Substrates for CD36 are unknown. It may be possible that CD36 regulates autophosphorylation of residue Thr92. Enzymes which modify CD36 are unknown. It may be possible that Thr92 is phosphorylated by extracellular threonine kinase(s).
Intracellular signalling is probably associated with phosphorylation of Fyn, Lyn and Yes, but the manner by which the cytoplasmic tail interacts with these PTKs is unknown.

### Main cellular expression of CD36:

CD36 is expressed on platelets, mature monocytes and macrophages, microvascular endo-thelial cells, mammary endothelial cells, during stages of erythroid cell development and on some macrophage derived dendritic cells, muscle cells, liver cells, and adipocytes.
The physiological events regulated by CD36 ligation are still very much unknown. Up to 50% of oxidized LDL are ingested through CD36, thus CD36 appears to be a major scavenger receptor. However, given the apparent absence of disease states in CD36 deficient subjects, other mechanisms appear to be capable of compensating for its absence.

### CD36 sequences

The CD36 polypeptide and/or CD36 nucleic acid molecule assessed by the method according preferably have one of the sequences shown below. Furthermore, a nucleic acid molecule may be a RNA nucleic acid molecule being complementary to one or more of the sequences shown below:
Gene Symbol CD36 HGNC
   Chromosomal Location 7q11.2
   UniGene ID
   Build 191 (07 May 2006)
   Hs.120949 NCBI (FULL LENGTH)
   Hs.248425
   Hs.75613
   Hs.325823
Entrez Gene 948 Entrez gene
   HGNC:1663
   HPRD: 01430
**SwissProt**
   P16671 EMBL-EBI
**OMIM**
   173510 NCBI
   248310 NCBI
   608404 NCBI
**Reference Sequences**
   ***Protein ID***
   NP_000063.2 NCBI
   NP_001001547.1 NCBI
   NP_001001548.1 NCBI
***Transcript ID***
   NM_000072 NCBI
   NM_001001547 NCBI
   NM_001001548 NCBI
**Nucleotide Sequence:**
   (SEQ ID NO.: 1)
   NM_001001548 2338 bp mRNA linear PRI 20-JAN-2008
   Homo sapiens CD36 molecule (thrombospondin receptor) (CD36), transcript variant 1, mRNA.
**ACCESSION NM_001001548 (VERSION NM_001001548.1)**
   ORIGIN 
1 gaggactgca gtgtaggact ttcctgcaga ataccatttg atcctattaa gaattgtcca
61 aatgttggag catttgattg aaaaatcctt cttagccatt ttaaagatag ctttccaatg
121 attagacgaa ttgattcttt ctgtgactca tcagttcatt tcctgtaaaa ttcatgtctt
 
181 gctgttgatt tgtgaataag aaccagagct tgtagaaacc actttaatca tatccaggag
241 tttgcaagaa acaggtgctt aacactaatt cacctcctga acaagaaaaa tgggctgtga
301 ccggaactgt gggctcatcg ctggggctgt cattggtgct gtcctggctg tgtttggagg
361 tattctaatg ccagttggag acctgcttat ccagaagaca attaaaaagc aagttgtcct
421 cgaagaaggt acaattgctt ttaaaaattg ggttaaaaca ggcacagaag tttacagaca
481 gttttggatc tttgatgtgc aaaatccaca ggaagtgatg atgaacagca gcaacattca
541 agttaagcaa agaggtcctt atacgtacag agttcgtttt ctagccaagg aaaatgtaac
601 ccaggacgct gaggacaaca cagtctcttt cctgcagccc aatggtgcca tcttcgaacc
661 ttcactatca gttggaacag aggctgacaa cttcacagtt ctcaatctgg ctgtggcagc
721 tgcatcccat atctatcaaa atcaatttgt tcaaatgatc ctcaattcac ttattaacaa
781 gtcaaaatct tctatgttcc aagtcagaac tttgagagaa ctgttatggg gctataggga
841 tccatttttg agtttggttc cgtaccctgt tactaccaca gttggtctgt tttatcctta
901 caacaatact gcagatggag tttataaagt tttcaatgga aaagataaca taagtaaagt
961 tgccataatc gacacatata aaggtaaaag gaatctgtcc tattgggaaa gtcactgcga
1021 catgattaat ggtacagatg cagcctcatt tccacctttt gttgagaaaa gccaggtatt
1081 gcagttcttt tcttctgata tttgcaggtc aatctatgct gtatttgaat ccgacgttaa
1141 tctgaaagga atccctgtgt atagatttgt tcttccatcc aaggcctttg cctctccagt
1201 tgaaaaccca gacaactatt gtttctgcac agaaaaaatt atctcaaaaa attgtacatc
1261 atatggtgtg ctagacatca gcaaatgcaa agaagggaga cctgtgtaca tttcacttcc
1321 tcattttctg tatgcaagtc ctgatgtttc agaacctatt gatggattaa acccaaatga
1381 agaagaacat aggacatact tggatattga acctataact ggattcactt tacaatttgc
1441 aaaacggctg caggtcaacc tattggtcaa gccatcagaa aaaattcaag tattaaagaa
1501 tctgaagagg aactatattg tgcctattct ttggcttaat gagactggga ccattggtga
1561 tgagaaggca aacatgttca gaagtcaagt aactggaaaa ataaacctcc ttggcctgat
1621 agaaatgatc ttactcagtg ttggtgtggt gatgtttgtt gcttttatga tttcatattg
1681 tgcatgcaga tcgaaaacaa taaaataaac ctggctcaag cacaaaccaa tttgtgttgt
1741 tctgattcaa taattggttt ctgggtggcc aattcagaag aagagtgtac atgctcaaca
1801 aatcctaggc cctgcattcc tgtcatcctc atccggggga aacaccatca tcccagtagc
1861 tgccctattc aactgcaaca gtctccagga ccatcagtat actgcatttc atgtgcacca
1921 aatattttga aagacattta taaataattg gcttatgact catatttctc tatgaatacc
1981 ttcatacagc aggtataact cttttcttta tgggcttaaa tattttgtca ctgatcctgc
2041 aaatggacat cattttagca cactagcggt ttatatttta aggaccttca ttctctgttc
2101 tgcacctctt ctggaaattg agtaaatttt gctttttttt ttttactcag ttgcaactta
2161 cgcttggcat cttcagaatg cttttctagc attaagagat gtaaatgata aaggaattat
2221 tgtatgaaat attacaaagc gtagactatg cattgttatt cattataata tttttgctg
2281 tcataatcgc ctcataaaga caggtttcaa ccattaaaat atgttcttcc ttaaaaaa
//
**ACCESSION NM_000072 (VERSlON NM_000072.2)** (SEQ ID NO.: 2)
   NM_000072 1983 bp mRNA linear PRI 20-JAN-2008
   DEFINITION Homo sapiens CD36 molecule (thrombospondin receptor) (CD36), transcript variant 3, mRNA.
ORIGIN 
1 gaggactgca gtgtaggact ttcctgcaga ataccatttg atcctattaa gaattgtcca
61 aatgttggag catttgattg aaaaatcctt cttagccatt ttaaagatag ctttccaatg
121 attagacgaa ttgattcttt ctgtgactca tcagttcatt tcctgtaaaa ttcatgtctt
181 gctgttgatt tgtgaataag aaccagagct tgtagaaacc actttaatca tatccaggag
241 tttgcaagaa acaggtgctt aacactaatt cacctcctga acaagaaaaa tgggctgtga
301 ccggaactgt gggctcatcg ctggggctgt cattggtgct gtcctggctg tgtttggagg
361 tattctaatg ccagttggag acctgcttat ccagaagaca attaaaaagc aagttgtcct
421 cgaagaaggt acaattgctt ttaaaaattg ggttaaaaca ggcacagaag tttacagaca
481 gttttggatc tttgatgtgc aaaatccaca ggaagtgatg atgaacagca gcaacattca
541 agttaagcaa agaggtcctt atacgtacag agttcgtttt ctagccaagg aaaatgtaac
601 ccaggacgct gaggacaaca cagtctcttt cctgcagccc aatggtgcca tcttcgaacc
661 ttcactatca gttggaacag aggctgacaa cttcacagtt ctcaatctgg ctgtggcagc
721 tgcatcccat atctatcaaa atcaatttgt tcaaatgatc ctcaattcac ttattaacaa
 
781 gtcaaaatct tctatgttcc aagtcagaac tttgagagaa ctgttatggg gctataggga
841 tccatttttg agtttggttc cgtaccctgt tactaccaca gttggtctgt tttatcctta
901 caacaatact gcagatggag tttataaagt tttcaatgga aaagataaca taagtaaagt
961 tgccataatc gacacatata aaggtaaaag gaatctgtcc tattgggaaa gtcactgcga
1021 catgattaat ggtacagatg cagcctcatt tccacctttt gttgagaaaa gccaggtatt
1081 gcagttcttt tcttctgata tttgcaggtc aatctatgct gtatttgaat ccgacgttaa
1141 tctgaaagga atccctgtgt atagatttgt tcttccatcc aaggcctttg cctctccagt
1201 tgaaaaccca gacaactatt gtttctgcac agaaaaaatt atctcaaaaa attgtacatc
1261 atatggtgtg ctagacatca gcaaatgcaa agaagggaga cctgtgtaca tttcacttcc
1321 tcattttctg tatgcaagtc ctgatgtttc agaacctatt gatggattaa acccaaatga
1381 agaagaacat aggacatact tggatattga acctataact ggattcactt tacaatttgc
1441 aaaacggctg caggtcaacc tattggtcaa gccatcagaa aaaattcaag tattaaagaa
1501 tctgaagagg aactatattg tgcctattct ttggcttaat gagactggga ccattggtga
1561 tgagaaggca aacatgttca gaagtcaagt aactggaaaa ataaacctcc ttggcctgat
1621 agaaatgatc ttactcagtg ttggtgtggt gatgtttgtt gcttttatga tttcatattg
1681 tgcatgcaga tcgaaaacaa taaaataagt aagtatgtac caaaaaatat tgcttcaata
1741 atattagctt atatattact tgttttcact ttatcaaaga gaagttacat attaggccat
1801 atatatttct agacatgtct agccactgat catttttaaa tataggtaaa taaacctata
1861 aatattatca cgcagatcac taaagtatat ctttaattct gggagaaatg agataaaaga
1921 tgtacttgtg accattgtaa caatagcaca aataaagcac ttgtgccaaa gttgtccaaa
1981 aaa
**ACCESSION NM_001001547 (VERSION NM_001001547.1) (SEQ ID NO.: 3)**
NM_001001547 2050 bp mRNA linear PRI 20-JAN-2008 Homo sapiens CD36 molecule (thrombospondin receptor) (CD36), transcript variant 2, mRNA.
ORIGIN 
1 agatgtcagg ataaccttaa ggatagatga agggttgaga gcctgtgcct catttctgag
61 ttctcagctg ctatgccgtg gaaatcctgt ttactttctg catctgctcc tgcaagactc
121 tggagccagt cttgaggtcc tacatctccg aaagcaagct cttctagaag ttgatagctt
181 tccaatgatt agacgaattg attctttctg tgactcatca gttcatttcc tgtaaaattc
241 atgtcttgct gttgatttgt gaataagaac cagagcttgt agaaaccact ttaatcatat
301 ccaggagttt gcaagaaaca ggtgcttaac actaattcac ctcctgaaca agaaaaatgg
361 gctgtgaccg gaactgtggg ctcatcgctg ggctgtcatt tggtgctgtc ctggctgtgt
421 ttggaggtat tctaatgcca gttggagacc tgcttatcca gaagacaatt aaaaagcaag
481 ttgtcctcga agaaggtaca attgctttta aaaattgggt taaaacaggc acagaagttt
541 acagacagtt ttggatcttt gatgtgcaaa atccacagga agtgatgatg aacagcagca
601 acattcaagt taagcaaaga ggtccttata cgtacagagt tcgttttcta gccaaggaaa
661 atgtaaccca ggacgctgag gacaacacag tctctttcct gcagcccaat ggtgccatct
721 tcgaaccttc actatcagtt ggaacagagg ctgacaactt cacagttctc aatctggctg
781 tggcagctgc atcccatatc tatcaaaatc aatttgttca aatgatcctc aattcactta
841 ttaacaagtc aaaatcttct atgttccaag tcagaacttt gagagaactg ttatggggct
901 atagggatcc atttttgagt ttggttccgt accctgttac taccacagtt ggtctgtttt
961 atccttacaa caatactgca gatggagttt ataaagtttt caatggaaaa gataacataa
1021 gtaaagttgc cataatcgac acatataaag gtaaaaggaa tctgtcctat tgggaaagtc
1081 actgcgacat gattaatggt acagatgcag cctcatttcc accttttgtt gagaaaagcc
1141 aggtattgca gttcttttct tctgatattt gcaggtcaat ctatgctgta tttgaatccg
1201 acgttaatct gaaaggaatc cctgtgtata gatttgttct tccatccaag gcctttgcct
1261 ctccagttga aaacccagac aactattgtt tctgcacaga aaaaattatc tcaaaaaatt
1321 gtacatcata tggtgtgcta gacatcagca aatgcaaaga agggagacct gtgtacattt
1381 cacttcctca ttttctgtat gcaagtcctg atgtttcaga acctattgat ggattaaacc
1441 caaatgaaga agaacatagg acatacttgg atattgaacc tataactgga ttcactttac
1501 aatttgcaaa acggctgcag gtcaacctat tggtcaagcc atcagaaaaa attcaagtat
1561 taaagaatct gaagaggaac tatattgtgc ctattctttg gcttaatgag actgggacca
1621 ttggtgatga gaaggcaaac atgttcagaa gtcaagtaac tggaaaaata aacctccttg
 
1681 gcctgataga aatgatctta ctcagtgttg gtgtggtgat gtttgttgct tttatgattt
1741 catattgtgc atgcagatcg aaaacaataa aataagtaag tatgtaccaa aaaatattgc
1801 ttcaataata ttagcttata tattacttgt tttcacttta tcaaagagaa gttacatatt
1861 aggccatata tatttctaga catgtctagc cactgatcat ttttaaatat aggtaaataa
1921 acctataaat attatcacgc agatcactaa agtatatctt taattctggg agaaatgaga
1981 taaaagatgt acttgtgacc attgtaacaa tagcacaaat aaagcacttg tgccaaagtt
2041 gtccaaaaaa
//
Protein sequence:
   (SEQ ID NO.: 4)
**ACCESSION NP_001001548(VERSION NP_001001548.1)**
ORIGIN 
1 mgcdmcgli agavigavla vfggilmpvg dlliqktikk qvvleegtia fknwvktgte
61 vyrqfwifdv qnpqevmmns sniqvkqrgp ytyrvrflak envtqdaedn tvsflqpnga
121 ifepslsvgt eadnftvlnl avaaashiyq nqfvqmilns linkskssmf qvrtlrellw
181 gyrdpflslv pypvtttvgl fypynntadg vykvfngkdn iskvaiidty kgkmlsywe
241 shcdmingtd aasfppfvek sqvlqffssd icrsiyavfe sdvnlkgipv yrfvlpskaf
301 aspvenpdny cfctekiisk nctsygvldi skckegrpvy islphflyas pdvsepidgl
361 npneeehrty ldiepitgft lqfakrlqvn llvkpsekiq vlknlkrnyi vpilwlnetg
421 tigdekanmf rsqvtgkinl lgliemills vgvvmfvafm isycacrskt ik

(SEQ ID NO.: 5)
 








**ACCESSION NP_000063 (VERSION NP_000063.2) (SEQ ID NO.: 6)**
ORIGIN 
1 mgcdmcgli agavigavla vfggilmpvg dlliqktikk qvvleegtia fknwvktgte
61 vyrqfwifdv qnpqevmmns sniqvkqrgp ytyrvrflak envtqdaedn tvsflqpnga
121 ifepslsvgt eadnftvlnl avaaashiyq nqfvqmilns linkskssmf qvrtlrellw
181 gyrdpflslv pypvtttvgl fypynntadg vykvfngkdn iskvaiidty kgkmlsywe
241 shcdmingtd aasfppfvek sqvlqffssd icrsiyavfe sdvnlkgipv yrfvlpskaf
301 aspvenpdny cfctekiisk nctsygvldi skckegrpvy islphflyas pdvsepidgl
361 npneeehrty ldiepitgft lqfakrlqvn llvkpsekiq vlknlkrnyi vpilwlnetg
421 tigdekanmf rsqvtgkinl lgliemills vgvvmfvafm isycacrskt ik

(SEQ ID NO.: 7)
 






 


**ACCESSION NP_001001547 (VERSION NP_001001547.1) (SEQ ID NO.: 8)**
ORIGIN

1 mgcdmcgli agavigavla vfggilmpvg dlliqktikk qvvleegtia fknwvktgte
61 vyrqfwifdv qnpqevmmns sniqvkqrgp ytyrvrflak envtqdaedn tvsflqpnga
121 ifepslsvgt eadnftvlnl avaaashiyq nqfvqmilns linkskssmf qvrtlrellw
181 gyrdpflslv pypvtttvgl fypynntadg vykvfngkdn iskvaiidty kgkrnlsywe
241 shcdmingtd aasfppfvek sqvlqffssd icrsiyavfe sdvnlkgipv yrfvlpskaf
301 aspvenpdny cfctekiisk nctsygvldi skckegrpvy islphflyas pdvsepidgl
361 npneeehrty ldiepitgft lqfakrlqvn llvkpsekiq vlknlkrnyi vpilwlnetg
421 tigdekanmf rsqvtgkinl lgliemills vgvvmfvafm isycacrskt ik

(SEQ ID NO.: 9)










### Sequence identity

Functional equivalents and variants are used interchangeably herein. In one preferred embodiment of the invention there is also provided variants of CD36 and variants of fragments thereof. When being polypeptides, variants are determined on the basis of their degree of identity or their homology with a predetermined amino acid sequence, said predetermined amino acid sequence being one of SEQ ID NO: 4, SEQ ID NO: 5, SEQ ID NO: 6, SEQ ID NO: 7, SEQ ID NO: 8 or SEQ ID NO: 9 when the variant is a fragment, a fragment of any of the aforementioned amino acid sequences, respectively.

Accordingly, variants preferably have at least 91 % sequence identity, for example at least 91% sequence identity, such as at least 92 % sequence identity, for example at least 93 % sequence identity, such as at least 94 % sequence identity, for example at least 95 % sequence identity, such as at least 96 % sequence identity, for example at least 97% sequence identity, such as at least 98 % sequence identity, for example 99% sequence identity with the predetermined sequence.

The following terms are used to describe the sequence relationships between two or more polynucleotides: "predetermined sequence", "comparison window", "sequence identity", "percentage of sequence identity", and "substantial identity".

A "predetermined sequence" is a defined sequence used as a basis for a sequence comparision; a predetermined sequence may be a subset of a larger sequence, for example, as a segment of a full-length DNA, transcriptional product thereof, gene sequence given in a sequence listing, such as a polynucleotide sequence of SEQ ID NO:1, SEQ ID NO: 2 or SEQ ID NO: 3 or may comprise a complete DNA or gene sequence. Generally, a predetermined sequence is at least 20 nucleotides in length, frequently at least 25 nucleotides in length, and often at least 50 nucleotides in length. Since two polynucleotides may each (1) comprise a sequence (i.e., a portion of the complete polynucleotide sequence) that is similar between the two polynucleotides, and (2) may further comprise a sequence that is divergent between the two polynucleotides, sequence comparisons between two (or more) polynucleotides are typically performed by comparing sequences of the two polynucleotides over a "comparison window" to identity and compare local regions of sequence similarity. A "comparison window", as used herein, refers to a conceptual segment of at least 20 contiguous nucleotide positions wherein a polynucleotide sequence may be compared to a predetermined sequence of at least 20 contiguous nucleotides and wherein the portion of the polynucleotide sequence in the comparison window may comprise additions or deletions (i.e., gaps) of 20 percent or less as compared to the predetermined sequence (which does not comprise additions or deletions) for optimal alignment of the two sequences.

Optimal alignment of sequences for aligning a comparison window may be conducted by the local homology algorithm of Smith and Waterman (1981) Adv. Appl. Math. 2: 482, by the homology alignment algorithm of Needleman and Wunsch (1970) J. Mol. Biol. 48: 443, by the search for similarity method of Pearson and Lipman (1988) Proc. Natl. Acad. Sci. (U.S.A.) 85: 2444, by computerized implementations of these algorithms (GAP, BESTFIT, FASTA, and TFASTA in the Wisconsin Genetics Software Package Release 7.0, Genetics Computer Group, 575 Science Dr., Madison, Wis.), or by inspection, and the best alignment (i.e., resulting in the highest percentage of homology over the comparison window) generated by the various methods is selected.

The term "sequence identity" means that two polynucleotide sequences are identical (i.e., on a nucleotide-by-nucleotide basis) over the window of comparison. The term "percentage of sequence identity" is calculated by comparing two optimally aligned sequences over the window of comparison, determining the number of positions at which the identical nucleic acid base (e.g., A, T, C, G, U, or I) occurs in both sequences to yield the number of matched positions, dividing the number of matched positions by the total number of positions in the window of comparison (i.e., the window size), and multiplying the result by 100 to yield the percentage of sequence identity. The terms "substantial identity" as used herein denotes a characteristic of a polynucleotide sequence, wherein the polynucleotide comprises a sequence that has at least 85 percent sequence identity, preferably at least 90 to 95 percent sequence identity, more usually at least 99 percent sequence identity as compared to a predetermined sequence over a comparison window of at least 20 nucleotide positions, frequently over a window of at least 25-50 nucleotides, wherein the percentage of sequence identity is calculated by comparing the predetermined sequence to the polynucleotide sequence which may include deletions or additions which total 20 percent or less of the predetermined sequence over the window of comparison. The predetermined sequence may be a subset of a larger sequence, for example, as a segment of the full-length SEQ ID NO:1, SEQ ID NO:2 and/or SEQ ID NO:3 polynucleotide sequence illustrated herein.

By the term "transcriptional or translational products" is meant herein products of gene transcription, such as a RNA transcript, for example an unspliced RNA transcript, a mRNA transcript and said mRNA transcript splicing products, and products of gene translation, such as polypeptide(s) translated from any of the gene mRNA transcripts and various products of post-translational processing of said polypeptides, such as the products of post-translational proteolytic processing of the polypeptide(s) or products of various post-translational modifications of said polypeptide(s).
As used herein, the term "transcriptional product of the gene" refers to a pre-messenger RNA molecule, pre-mRNA, that contains the same sequence information (albeit that U nucleotides replace T nucleotides) as the gene, or mature messenger RNA molecule, mRNA, which was produced due to splicing of the pre-mRNA, and is a template for translation of genetic information of the gene into a protein.
As used herein, the term "translational product of the gene" refers to a protein, which is encoded by the CD36 gene.

As used herein, the term "transcriptional product of the gene" refers to a transcript which is encoded by the CD36 encoding gene (SEQ ID NO:1, SEQ ID NO:2, SEQ ID NO:3). It is appreciated by the person skilled in the art that a number of isoforms of CD36 is known. Isoforms are versions of a protein with some small differences, usually a splice variant or the product of some posttranslational modification. The present invention relates to any isoform of CD36. The examples given herein are not meant to be limiting to the scope of the present invention.

Thus the present disclosure relates to methods for classifying, diagnosing and/or monitoring the atherosclerotic plaques in an individual by for example determining the concentration of at least one CD36 transcript corresponding to the transcriptional product of SEQ ID NO:1, SEQ ID NO:2, and/or SEQ ID NO:3, or part thereof.

In particular, the disclosure relates to methods involving the steps of determining the concentration of a CD36 encoding nucleic acid molecule, being the transcriptional products of the CD36 encoding gene in
(i) a nucleic acid sequence identified in the invention as SEQ ID NO:1, SEQ ID NO:2, and/or SEQ ID NO:3 or fragments thereof,
(ii) a nucleic acid sequence having at least 90% identity with SEQ ID NO: 1, SEQ ID NO:2, and/or SEQ ID NO:3 or fragments thereof,
(iii) a nucleic acid sequence complementary to any of the sequences of (i) or (ii),

The invention also relates to methods involving the step of determining the concentration of a CD36 polypeptide or part thereof in
(i) variant proteins corresponding to the protein identified as SEQ ID NO: 4, SEQ ID NO: 5, SEQ ID NO: 6, SEQ ID NO: 7, SEQ ID NO: 8 or SEQ ID NO: 9 or variants, or fragments thereof,
(ii) polypeptide sequences having at least 90% identity with the variant proteins, or fragments thereof, of (i),

Thus, it is an embodiment of the disclosure to use the above identified variant proteins for the purpose of
i) classifying atherosclerotic plaques in an individual
ii) diagnosing atherosclerotic plaques in an individual
iii) monitoring atherosclerotic plaques in an individual
iv) diagnosing an individual at risk of having and/or acquiring a symptomatic atherosclerotic plaques
v) diagnosing burden of atherosclerotic plaques in an individual
vi) diagnosing stenosis caused by atherosclerotic plaques in an individual and/or
vii) determining the treatment regime of an individual

Sequence identity is determined in one embodiment by utilising fragments of CD36 peptides comprising at least 25 contiguous amino acids and having an amino acid sequence which is at least 80%, such as 85%, for example 90%, such as 95%, for example 99% identical to the amino acid sequence of SEQ ID NO: 4, SEQ ID NO: 5, SEQ ID NO: 6, SEQ ID NO: 7, SEQ ID NO: 8 or SEQ ID NO: 9, wherein the percent identity is determined with the algorithm GAP, BESTFIT, or FASTA in the Wisconsin Genetics Software Package Release 7.0, using default gap weights.

### Conservative amino acid substitutions:

Substitutions within the groups of amino acids, shown below, are considered conservative amino acid substitutions. Substitutions between the different groups of amino acids are considered non-conservative amino acid substitutions.
P, A, G, S, T (neutral, weakly hydrophobic)
Q, N, E, D, B, Z (hydrophilic, acid amine)
H, K, R (hydrophilic, basic)
F, Y, W (hydrophobic, aromatic)
L, I, V, M (hydrophobic)
C (cross-link forming)

### Individuals

The present invention relates to individuals, for example mammals and in particular humans of any sex or age. The individuals are asymptomatic with regard to the diseases caused by artherosclerosis as described herein. Thus, the individual of the present invention may never have suffered from any of atherosclerosis-related diseases. However, it is also within the scope of the present invention that the individual of the present invention may previously have been suffering from atherosclerosis-related diseases but are asymptomatic at the time for carrying out the methods of the present invention.

### Sample

The sample according to the disclosure may be a body fluid sample, in particular a cell-free plasma or serum sample. That the plasma is cell-free may be verified microscopically following centrifugation of the blood sample. Centrifugation causes the blood cells to be separated from the plasma. Centrifugation of a blood sample in the range of 2,500 to 3000G will result in a cell-free plasma. In one embodiment the sample is a peripheral venous blood sample. A preferred embodiment is a cell-free sample, preferably a cell-free plasma sample.

Suitable plasma preparations may be plasma from heparin-stabilised blood, Citrate-stabilised or EDTA-stabilised blood.

The samples may be fresh or frozen. For example the samples are frozen at -80°C and are thawed only once prior to determining the concentration of CD36 and/or CD36 encoding nucleic acid.

### Immunological methods

In the context of the present invention, "immunological methods" are understood as meaning analytical methods based on immunochemistry, in particular on an antigen-antibody reaction. Examples of immunological methods include immunoassays such as radioimmunoassay (RIA), enzyme immunoassay (EIA, combined with solid-phase technique: ELISA (Enzyme Linked Immuno Sorbent Assay)) or else immunofluorescence assays. The immunoassay is carried out by exposing the sample to be investigated to a CD36-binding antibody and detecting and quantifying the amount of CD36 bound to this antibody. In these assays, detection and quantification is carried out directly or indirectly in a known manner. Thus, detection and quantification of the antigen-antibody complexes is made possible by using suitable labels which may be carried by the antibody directed against CD36 and/or by a secondary antibody directed against the primary antibody. Depending on the type of the abovementioned immunoassays, the labels are, for example, radioactive labels, a chemiluminescent label, fluorescent dyes or else enzymes, such as phosphatase or peroxidase, which can be detected and quantified with the aid of a suitable substrate.

In one embodiment of the invention, the immunological method is carried out with the aid of a suitable solid phase. Suitable solid phases which may be mentioned include the customary commercial microtiter plates made of polystyrene or membranes (for example made of polyvinylidene difluoride, PVDF) which are customarily used for the ELISA technique.

To carry out a process according to the invention, a suitable sample, such as a liquid patient sample is applied to the solid phase. The sample is preferably a plasma sample wherein the CD36 or fraction thereof is present in unbound form or is present in a form which could be bound to its ligand LDL. The assumption herein that sCD36 is present in complex with a high molecular fraction in plasma implies that it is preferred to freeze and thaw the samples to be tested, such that said high molecular complex (lipid-protein complex) possibly is degraded.

In one aspect the invention relates to a method, wherein the CD36 polypeptide concentration is determined by i) providing a sample to be investigated, ii) providing an anti-CD36 antibody, iii) exposing the sample to the anti-CD36 antibody, iv) optionally, exposing said CD36-antibody complex to at least a further antibody directed against said CD36-antibody complex, and v) detecting and quantifying the amount of said antibody

In a more specific aspect of the invention there is provided a method for determining human CD36 in a plasma sample by solid phase ELISA enzyme immunoassay which comprises the steps of
(i) providing a plasma sample to be investigated,
(ii) providing an anti-CD36 antibody as defined herein,
(iii) exposing the sample to be investigated to a solid phase and the antibody, and
(iv) detecting and quantifying the amount of the antibody which binds to CD36; and
in a more preferred aspect of the invention there is provided a method for determining human CD36 in a plasma sample by solid phase ELISA enzyme immunoassay which comprises the steps of
(i) providing a plasma sample to be investigated,
(ii) providing an anti-CD36 antibody,
(iii) exposing the sample to be investigated to the anti-CD36 antibody bound to a solid phase,
(iv) optionally exposing the CD36-antibody complex to a second anti-CD36 antibody, and
(iv) detecting and quantifying the amount of the antibody which binds to CD36,
and wherein the solid phase preferably is a microtiter plate, the CD36 is preferably present (part of) in a high molecular weight plasma fraction said high molecular weight plasma fraction preferably being a CD36-lipoprotein complex; said anti-CD36 antibody is preferably selected from the group consisting of monoclonal antibodies as specified below; said determination is preferably carried out by a solid phase enzyme immunoassay, wherein, in the enzyme immunoassay, the sample is exposed to a first, human CD36-binding antibody, and the amount of bound CD36 is measured using a second CD36-binding antibody carrying an enzyme label, where the measurement is carried out by an enzyme-catalyzed colour reaction or chemiluminescence.

It is also within the scope of the present invention to determine the concentration of CD36 by multiplex suspension array technique.

### Antibodies

One aspect of the present disclosure relates to an antibody directed to an epitope of CD36 polypeptide or part thereof as described elsewhere herein. The antibody may be used in methods of the present invention relating to methods for classifying an atherosclerotic plaque, diagnosing an atherosclerotic plaque, monitoring an atherosclerotic plaque, diagnosing a risk of having and/or acquiring a symptomatic atherosclerotic plaque, diagnosing the burden of an atherosclerotic plaque, diagnosing stenosis, and/or determining the treatment regime of an individual. Thus, epitope in this context covers any epitope capable of being recognized by an antibody or a binding fragment thereof.

The term "antibody" as used herein includes both polyclonal and monoclonal antibodies, as well as fragments thereof, such as, Fv, Fab and F(ab)2 fragments that are capable of binding antigen or hapten. It includes conventional murine monoclonal antibodies as well as human antibodies, and humanized forms of non-human antibodies, and it also includes 'antibodies' isolated from phage antibody libraries.

The antibodies may be polyclonal or monoclonal and may be produced by in vivo or in vitro methods known in the art. Thus, the anti-CD36 antibody is selected from the group consisting of monoclonal and polyclonal CD36-specific antibodies.

A monoclonal antibody is an antibody produced by a hybridoma cell. Methods of making monoclonal antibody-synthesizing hybridoma cells are well known to those skilled in the art, e.g, by the fusion of an antibody producing B lymphocyte with an immortalized B-lymphocyte cell line.

A polyclonal antibody is a mixture of antibody molecules (specific for a given antigen) that has been purified from an immunized (to that given antigen) animal's blood, where a non-limiting example is antibody molecules from rabbit. Such antibodies are polyclonal in that they are the products of many different populations of antibody-producing cells.

The disclosure also pertains to mixtures of monoclonal and/or polyclonal antibodies. Also a mixture of at least two monoclonal antibodies is within the scope of the present invention. It is appreciated that the mixture may comprise 3, 4, 5, 6, 7, 8, 9, 10, or 15 monoclonal antibodies.

The anti-CD36 antibody used herein may be a monoclonal antibody as described above, and/or an isolated polyclonal antibody, obtainable by an immunization process in which purified or recombinant human CD36 is used as antigen component and the antibody is preferably specific for various fractions of the CD36 protein which is present in human blood plasma in soluble form, optionally as a part of a CD36-lipoprotein complex, such as CD36 or a fraction thereof bound to Low Density Lipoprotein (LDL), IDL (Intermediate Density Lipoprotein or VLDL (Very Low Density Lipoprotein) which may be present in a high molecular weight fraction of cell-free plasma. In one embodiment the CD36-lipoprotein complex comprises oxidized forms of lipoproteins. For example, sCD36 is associated with oxidized LDL (oxLDL), oxidized LDL, oxidized IDL and/or oxidized VLDL. In yet another embodiment sCD36 is found in complex with oxidized HDL.

In one embodiment the antibody is selected from the group consisting of sc7309 (CD36 (SMf), mouse IgM) , and polyclonal CD36 specific antibodies, such as sc5522 (CD36 (N-15), goat IgG, epitope N-terminus (h)), sc9154 (CD36(H-300), rabbit IgG, epitope 1-300 (h)), such as sc5522 and sc9154 (both from Santa Cruz Biotechnology Inc., Santa Cruz, California, USA).

The antibodies used in the ELISA assay for detection of circulating CD36 in plasma are preferably labelled for ease of detection, the label preferably being in the form of a biotinylation which is well known to a person skilled in the art.

In particular the CD36-specific monoclonal antibody may be selected from the group consisting of:
185-1 G2 Vilella
131.1 Tandon, Rockville
131.2 Tandon, Rockville
131.4 Tandon, Rockville
131.5 Tandon, Rockville
131.7 Tandon, Rockville
NAM28-8C12 Blanchard, Nantes
AmAK-5 Kehrel, Muenster
CLB-IVC7 CLB, Amsterdam
Lyp 10.5 McGregor, Lyon
Lyp 13.10 McGregor, Lyon

### Standard level used in diagnosis, classification and monitoring

In an individual suffering from an atherosclerotic plaque an increase in the concentration of CD36 polypeptide of at least 1.15 times of the standard level is an indication of progression of the atherosclerotic plaque towards a symptomatic atherosclerotic plaque. In particular an increase of at least 1.25 of the standard level, for example an increase of at least 1.30, such as increase of at least 1.35 of the standard level, for example 1.40 of the standard level, such as increase of at least 1.45 of the standard level, for example 1.50 of the standard level, such as an increase of at least 1.55 of the standard level, such as an increase of at least 1.60 of the standard level, such as an increase of at least 1.65 of the standard level, such as an increase of at least 1.70 of the standard level, such as increase of at least 1.75 of the standard level, such as an increase of at least 1.80 of the standard level, such as an increase of at least 1.85 of the standard level, such as an increase of at least 2.0 of the standard level, such as an increase of at least 2.25 of the standard level, such as an increase of at least 2.50 of the standard level, such as an increase of at least 3 of the standard level, such as an increase of at least 3.50 of the standard level, such as an increase of at least 4 of the standard level, such as an increase of at least 4.50 of the standard level, such as an increase of at least 5 of the standard level, such as an increase of at least 6, 7, 8, 9 or 10 of the standard level is indicative of progression of the atherosclerotic plaque towards a symptomatic atherosclerotic plaque.

By the term "standard level" is meant the concentration of CD36 polypeptide in a pool of samples from a random group of individuals. In one embodiment the standard level is determined in a pool of sample from a random group of asymptomatic individuals. The pool comprises samples from at least 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or such as at least 100 individuals.

In another embodiment the standard level is the concentration of CD36 polypeptide in a pool of samples from a group of individuals such as at least 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or such as at least 100 individuals, in which no symptoms related to atherosclerosis have been observed more than 2 months ago, more than 3 months ago, more than 4 months ago, more than 5 months ago, or more than 6 months ago at the time for supplying a sample. In one embodiment the standard level is the concentration of CD36 polypeptide or CD36 encoding nucleic acid molecule in a pool of samples from a group of individuals such as at least 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or such as at least 100 individuals, in which no symptoms related to carotid stenosis and/or carotid sclerosis have been observed more than 2 months ago, more than 3 months ago, more than 4 months ago, more than 5 months ago, or more than 6 months ago at the time for supplying a sample.

### Kit

In one aspect the invention relates to a use of a kit for diagnosing atherosclerotic plaques in an individual, said method comprising in a sample from said individual i) determining the concentration of a CD36 polypeptide, ii) correlating said concentration determined in i) to a standard level, and iii) based on said correlation according to ii) diagnosing said atherosclerotic plaques.

In a further aspect the disclosure relates to a kit for diagnosing an individual at risk of having and/or acquiring a symptomatic atherosclerotic plaques, said method comprising in a sample from said individual, i) determining the concentration of a CD36 polypeptide, ii) correlating said concentration determined in i) to a standard level, and iii) based on said correlation according to ii) diagnosing whether said individual is at risk of having and/or acquiring a symptomatic atherosclerotic plaque.

Furthermore, the present invention relates to a use of a kit for diagnosing stenosis caused by atherosclerotic plaques in an individual, said method comprising in a sample from said individual i) determining the concentration of a CD36 polypeptide, ii) correlating said concentration determined in i) to a standard level, and iii) based on said correlation according to ii) diagnosing the degree of stenosis caused by atherosclerotic plaques in an individual.

The one or more kits thus comprises at least one detection member for determination of the concentration of a CD36 polypeptide.
In one embodiment the detection member is an antibody capable of specifically binding the CD36 polypeptide or part thereof.

The concentration of a CD36 polypeptide or fragment thereof can be compared manually by a person or by a computer or other machine. An algorithm can be used to detect similarities and differences. The algorithm may score and compare, for example, the genes which are expressed and the genes which are not expressed. Alternatively, the algorithm may look for changes in intensity of expression of a particular gene, transcript or translational product thereof and score changes in intensity between two samples. Similarities may be determined on the basis of genes, transcripts or translational products thereof which are expressed in both samples and genes which are not expressed in both samples or on the basis of genes whose intensity of expression are numerically similar.

Generally, the detection operation will be performed using a reader device external to the diagnostic device. However, it may be desirable in some cases to incorporate the data gathering operation into the diagnostic device itself.

The detection apparatus may be a fluorescence detector, or a spectroscopic detector, or another detector.

Although hybridization is one type of specific interaction which is clearly useful for use in this mapping embodiment antibody reagents may also be very useful.

Gathering data from the various analysis operations, e.g. oligonucleotide and/or micro-capillary arrays will typically be carried out using methods known in the art. For example, the arrays may be scanned using lasers to excite fluorescently labeled targets that have hybridized to regions of probe arrays mentioned above, which can then be imaged using charged coupled devices ("CCDs") for a wide field scanning of the array. Alternatively, another particularly useful method for gathering data from the arrays is through the use of laser confocal microscopy which combines the ease and speed of a readily automated process with high resolution detection.

Following the data gathering operation, the data will typically be reported to a data analysis operation. To facilitate the sample analysis operation, the data obtained by the reader from the device will typically be analyzed using a digital computer. Typically, the computer will be appropriately programmed for receipt and storage of the data from the device, as well as for analysis and reporting of the data gathered, i.e., interpreting fluorescence data to determine the sequence of hybridizing probes, normalization of background and single base mismatch hybridizations, ordering of sequence data in SBH applications, and the like.

### Experimentals and Examples

### Patients

Carotid plaques from consecutive endarterectomy patients were classified into two groups depending on whether or not the patients had experienced ipsilateral stroke, TIA, or amaurosis fugax in the prior six month to surgery. Plaques were characterized as symptomatic or asymptomatic according to the presence or absence of cerebrovascular symptoms, respectively. The carotid stenoses were diagnosed and classified by precerebral color Duplex ultrasound (Grant EG, Benson CB, Moneta GL, Alexandrov AV, Baker JD, Bluth EI, Carroll BA, Eliasziw M, Gocke J, Hertzberg BS, Katanick S, Needleman L, Pellerito J, Polak JF, Rholl KS, Wooster DL, Zierler RE. Carotid artery stenosis: gray-scale and Doppler US diagnosis-Society of Radiologists in Ultrasound Consensus Conference. Radiology. 2003; 229:340-346) and CT angiography (Anderson GB, Ashforth R, Steinke DE, Ferdinandy R, Findlay JM. CT angiography for the detection and characterization of carotid artery bifurcation disease. Stroke. 2000; 31:2168-2174) according to consensus criteria. The asymptomatic carotid stenoses were detected during clinical examinations of patients with coronary artery disease (CAD), peripheral artery disease, or stroke/TIA more than six month ago.

### Carotid endarterectomy specimens

Atherosclerotic carotid plaques were retrieved from patients during carotid endarterectomy. Plaques that were used for protein and RNA extraction were rapidly frozen in liquid nitrogen. For Western blots, the tissue powders from the plaques were homogenized in ice-cold lysis buffer (PBS containing protease inhibitor cocktail [Gibco, Paisley, UK] with 1% Triton X-100 and 0.1% Tween 20) at a ratio of 0.1 ml per 10 mg wet weight tissue by a metal blade homogenizer. Extracts were incubated on ice for 15 minutes, and centrifuged at 12 000g (15 minutes at 4°C). The supernatants were retained and protein concentrations in the samples were measured with the BCA method (Pierce, Cheshire, UK).

### Blood sampling protocol

Peripheral venous blood was drawn into pyrogen-free EDTA tubes that were immediately immersed in melting ice and centrifuged at 2,500g for 20 minutes within 20 minutes to obtain platelet-poor plasma. All samples were stored at -80°C and thawed only once before start of assay procedures.

### High-density oligonucleotide microarrays

Total RNA was isolated from frozen carotid tissue using MagNA Pure Kit III (Roche Applied Science, Indianapolis, IN), quantified spectrophotometrically, and stored at - 80°C. The Human Genome U 133A 2.0 Array encoding 14500 genes was purchased from Affymetrix (Santa Clara, CA), and hybridization was performed according to the manufacturer's two cycle target labelling protocol. Briefly, cDNA was prepared from 100 ng total RNA and cRNA was obtained from *in vitro* transcription of the cDNA. Then the cycle was repeated making cDNA from 600 ng cRNA. Thereafter biotin-labeled cRNA was generated from *in vitro* transcription of cDNA and fragmented before hybridization to the array. For data analyses, GeneChip Operation Software (1.3) and ArrayAssist (3.4) were used.

### Example 1

Example of ELISA analysis kit for detecting CD36 in serum
ELISA analysis, reagents used and assay conditions
Phosphate buffer 0,1 mol/l, pH 8,0
Na₂HPO4, 2H₂O, 0,1 mol/l, pH 8,0, stored at 4°C.
POD buffer
NaH₂PO4, H₂O, 1,5 mmol/l
Na₂HPO4, 2 H₂O, 8,5 mmol/I
NaCl 400 mmol/l
pH 7,4
Stored at 4°C 20
Preparation of lysozyme solution:
Lysozyme, Sigma L-6876, 20 mg/ml. Stored at 20°C in 1 ml portions, storage life 1 year. Storage life at 4°C is 4 days.
POD-avidin Dako P364: Colour reagent
TMB Microwell Peroxidase Substrate (1 component)
Cat. No. 50-76-06, Kirkegård and Perry Laboratories. Storage life 1 year at 2-25°C 25 Phosphoric acid, 1 mol/l
POD-avidin solution:

POD buffer 12 ml
Lysozyme solution 120µl 30
POD-avidin 6 µl
To be prepared immediately before use.
Antibody
Anti-CD36: biotinylated sc-9154 (rabbit polyclonal antibody from Santa Cruz)

### Apparatus

### Automatic microtiter plate-rinser EIx50 (Biotek Instruments).

ELISA plates were coated overnight at 4°C with catcher antibody goat (sc5522)-CD36 (0.1 µg/ml), followed by blocking with phosphate-buffered saline (PBS) and 0.1 %Tween for 2 days at 4°C. The plates were stored at -20°C until use. A microtiter plate coated with antibody against CD36 is rinsed 3 times with 340 µl rinsing buffer pr. well using program 12. The last rinse is terminated without aspiration of rinsing buffer, which is first decanted immediately before application of standards (EDTA-plasma pool) and samples (with possible controls), and immediately before addition of reagent 5

### Standards

EDTA blood from 100 subjects from the routine blood sampling are centrifuged at 3000 G for 10 min, the upper part of the plasma is pipetted off and pooled. Pool-plasma is frozen in aliquots of 350µl and dilutions are used as standard curves. Other EDTA plasma pools are used as high and low internal assay controls.
The intra assay coefficient of variation, which was estimated from a run of 76 single determinations of the same sample, was 10%, and estimated from double determinations of the high control on 15 different days was 6 %. The inter assay coefficient of variation, estimated from the controls in each run performed, was 16.4%. Runs were only accepted when controls were within the range of +/- 1.96 x SD (inter assay).

### Method for Elisa assay

A microtiter plate is rinsed on the EIx50. Standards, controls, samples and dilution buffer are applied in double rows, 100 µl/well. The positions of the applications are noted. The microtiter plate is covered with plastic film and incubated for 60 min. on a shaking table.
The plate is rinsed on the EIx50. 100 µl biotinylated sc-9154 is added per well, covered with plastic film and incubate 60 min on a shaking table. The plate is rinsed on the EIx50.
100 µl POD-avidin solution is added per well. Covered with plastic film and incubate for about 30 min. on a shaking table. The plate is rinsed on the Elx50. In a fume hood 100 µl TMB is added per well. Cover with plastic film, incubate for about 10 min. on a shaking table. The reaction is terminated with 100 µl phosphoric acid per well (in a fume hood). Cover with plastic film until reading.

### Measurement

Read the extinctions at 450 nm and 620 nm on a Multiscan apparatus before 60 min following the termination of the reaction.

### Calculation

Cubic spline with linear scale on both axes. Dilutions of EDTA pool are used as standard curve, and results are expressed relative to the EDTA plasma pool.
Elisa dilution buffer: phosphate buffer 10 mmol/l with 0,145 mol/l NaCl, pH 7,4

### Example 2

CD36 gene expression in atherosclerotic plaques

**Table 1. CD36 was differently expressed in plaques obtained from asymptomatic (n=4) compared to symptomatic (n=4) carotid atherosclerotic patient. 5 out of 5 probe sets on the Affimetrix (Human Genome U133A 2.0 Array) detected CD36 (Hs. 120949)**

| **UniGene ID** | **Gene Name** | **Gene Symbol** | **Ratio*** |
|---|---|---|---|
| Hs.120949 | CD36 antigen (collagen type I receptor thrombospondin receptor) | CD36 | 0.38 |
| Hs.120949 | CD36 antigen (collagen type I receptor thrombospondin receptor) | CD36 | 0.22 |
| Hs.120949 | CD36 antigen (collagen type I receptor thrombospondin receptor) | CD36 | 0.14 |
| Hs.120949 | CD36 antigen (collagen type I receptor thrombospondin receptor) | CD36 | 0.14 |
| Hs.120949 | CD36 antigen (collagen type I receptor thrombospondin receptor) | CD36 | 0.07 |

| | | | |
|---|---|---|---|
| * asymptomatic versus symptomatic | | | |

### Examples 3 to 5

### Patients

Sixty-two consecutive patients with high-grade internal carotid stenoses (≥ 70%) were treated with carotid endarterectomy (CEA, 53 patients) or carotid angioplasty with stenting (CAS, 9 patients) (Table 2). The study included 19 (31%) women and 43 (69 %) men, aged 65.5 years (range 44-83 years). The patients were classified into two groups according to their symptoms. Sixteen (26%) patients had suffered from clinical symptoms such as stroke, transitory ischemic attack (TIA) or amaurosis fugax ipsilateral to the stenotic internal carotid artery within the past 2 months whereas 46 (74%) patients had symptoms more than 2 months ago (n=22) or had never suffered from symptoms as outlined above (n=24) (Table 2). The carotid stenoses were diagnosed and classified using precerebral color Duplex and CT angiography according to consensus criteria (Anderson et al. Stroke. 2000; Grant EG et al. Radiology. 2003;229:340-346).

The asymptomatic carotid stenoses were detected during clinical examinations of patients with coronary artery disease (CAD), peripheral artery disease or stroke/TIA more than six months ago. The plaques were also divided into two groups, depending on plaque echogenicity on ultrasound examination according to methods described previously (European Carotid Plaque Study, Eur J Vasc Endovasc Surg. 1995;10: 23-30; Mathiesen EB et al, Circulation. 2001;103: 2171-2175), and classified as echolucent or echogenic/heterogeneous. Triplex ultrasound of precerebral arteries, clinical neurological examination, and cerebral diffusion-weighted magnetic resonance imaging (MRI) were performed within two days before and the day after the procedures. Patients with concomitant inflammatory diseases such as infections and autoimmune disorders, or liver or kidney disease were excluded from all parts of the study. The protocols were approved by the regional ethics committee and signed informed consent was obtained from all individuals.

**Table 2 Baseline variables in patients according to # clinical symptoms and months after symptoms (n=62)**

| | ≤ **2months (n=16)** | **>2months or no symptoms(n=46)** | **p** |
|---|---|---|---|
| Age, year | 66 (7.8) | 65 (9.3) | .98 |
| Male sex* | 12(75) | 31 (67) | .57 |
| Degree of stenoses, % | 80 (80-95) | 80 (70-95) | .65 |
| Echolucent carotid plaque* | 7 (43.8) | 13 (28.3) | .25 |
| Ischemia on cerebral MRI* (n=53) | 11 (68.8) | 31 (67.4) | .94 |
| Body mass index, kg/m2 | 26.7 (20.4-35.5) | 25.8 (19-35.5) | .72 |
| CEA treated* | 15 (93.8) | 38 (82.6) | .28 |
| Systolic blood pressure, mmHg | 151 (110-196) | 150 (110-194) | .60 |
| Diastolic blood pressure, mmHg | 73 (32-99) | 80 (49-101) | .59 |
| Diabetes mellitus* | 4 (25) | 10 (22) | .79 |
| Statin treatment* | 13 (81.3) | 41 (89.1) | .42 |
| Current smoking* | 7 (43.8) | 25 (54.3) | .74 |
| Neopterin, nmol/l | 8.7 (4.9-18.0) | 9.1 (5.7-60.3) | .48 |
| CRP, mg/l | 3.5 (1.0-39.0) | 5 (0.6-28.0) | .93 |
| Total leukocyte count, 10°/l | 8.3 (4.0-12.1) | 7.5 (3.8-11.1) | .31 |
| Fibrinogen, g/l | 4.0 (2.9-6.6) | 3.9 (2.6-6.9) | .87 |
| Cholesterol, mmol/l | 4.3 (3.6-5.9) | 4.4 (2.8-7.5) | .68 |
| HDL cholesterol, mmol/l | 1.3 (0.7-1.9) | 1.2 (0.8-2.5) | .82 |
| Triglycerides, mmol/l | 1.4 (0.6-3.8) | 1.5 (0.7-3.7) | .31 |
| LDL (n=49) | 2.6 (2.0-4.0) | 2.6 (1.5-5.1) | .46 |
| HbA1c, % | 5.7 (5.2-6.9) | 5.8 (0.9-8.8) | .95 |
| β-thromboglobulin, IU/ml | 53.3 (26.7-142.1) | 54.8 (15.4-268.5) | .61 |
| Platelet count, 10⁹/I | 225.5 (132-391) | 261 (168-441) | 10 |

| | | | |
|---|---|---|---|
| # Clinical symptoms include stroke, TIA or amaurosis fugax ipsilateral to the stenotic internal carotid artery. Numbers are un-adjusted means (SD) or *numbers (percentages) | | | |

### Blood sampling protocol

Peripheral venous blood was drawn into pyrogen-free EDTA tubes that were immediately immersed in melting ice and centrifuged at 2,500g for 25 minutes at 4°C within 20 minutes to obtain platelet-poor plasma. All samples were stored at -80°C and thawed only once.

### Immunohistochemistry

Acetone-fixed sections of atherosclerotic carotid plaques, retrieved from patients during carotid endarterectomy, were stained using monoclonal mouse anti-human CD36 (FA6-152, Novus Biologicals, Littleton, CO), anti-human CD3 IgG (DakoCytomation, Glostrup, Denmark), and anti-human smooth muscle actin (DakoCytomation), affinity purified polyclonal mouse anti-human macrophages (calprotectin) IgG (MCA874G, Serotec Ltd., Oxford, UK), and sheep anti-rat von Willebrand factor IgG (Cedarlane, Ontario, Canada). The primary antibodies were followed by biotinylated anti-mouse or anti-sheep IgG (Vector Laboratories, Burlingame, CA). The immunoreactivities were further amplified using avidin-biotin-peroxidase complexes (Vectastain Elite kit, Vector Laboratories). Diaminobenzidine was used as the chromogen in a commercial metal enhanced system (Pierce Chemical, Rockford, IL). The sections were counter-stained with hematoxylin. Omission of the primary antibody served as a negative control.

### Soluble CD36 enzyme-linked immunoassay (ELISA)

ELISA plates were coated overnight at 4°C with catcher antibody goat (sc5522)-CD36 (0.1 µg/ml), followed by blocking with phosphate-buffered saline (PBS) and 0.1 %Tween for 2 days at 4°C. The plates were stored at -20°C until use. Detection antibody sc9154 was biotinylated as described elsewhere (Glintborg D et al. Published online ahead of print November 13, 2007). All antibodies were from Santa Cruz Biotechnology (Santa Cruz, CA). A pool of EDTA-plasma from 100 subjects from routine blood sampling served as standard plasma, and was applied in increasing dilutions in duplicates. Other pools of EDTA-plasma served as high and low controls and PBS was used as background control. Patient samples were applied in appropriate dilutions in duplicates. Following 1 hour incubation at room temperature, the wells were rinsed, and biotinylated anti CD36 (sc9154) was applied and incubated for 30 minutes. After rinsing, TMB Microwell Peroxidase Substrate (KemEnTec, Copenhagen, Denmark) was added. The reaction was terminated by phosphorous acid, and extinctions were determined at 450 nm and 620 nm on a Multiscan Apparatus (Thermo, Langenselbold, Germany). Absorptions were calculated relative to the standard EDTA plasma pool and expressed as relative units. Controls were allowed to deviate by 1 SD from the true value. Intra-assay and long-term inter-assay coefficient of variation were 6% and 16%, respectively.

### Miscellaneous

C-reactive protein (CRP) levels were determined by a high-sensitivity particle enhanced immunoturbidimetric assay on a modular platform (Roche Diagnostic, Basel, Switzerland). Concentrations of lipid parameters and HbA1 c were measured by routine laboratory methods as previously described. Plasma levels of neopterin and β-thromboglobulin (⊃-TG) were measured by ELISAs obtained from IBL Hamburg (Hamburg, Germany) and Diagnostica Stago (Asnières, France), respectively.

### Statistical analyses

For comparisons of 2 groups of individuals, the Mann-Whitney U test was used. When comparing 3 groups of individuals, the non-parametric Kruskal-Wallis test was used. If a significant difference was found, Mann-Whitney U test was used to calculate the difference between each pair of groups. Coefficients of correlation (r) were calculated by the Spearman rank test. The Fisher's exact test was used when comparing proportions. The relationship between variables was calculated using the linear and binary regression analysis for continuous and categorical dependent variables, respectively. Probability values (2-sided) were considered significant at value of <0.05.

### Example 3

### Plasma CD36 and plaque stability

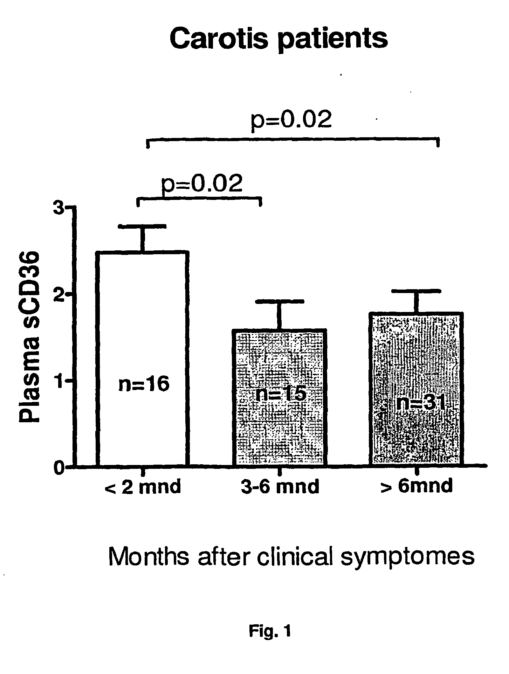

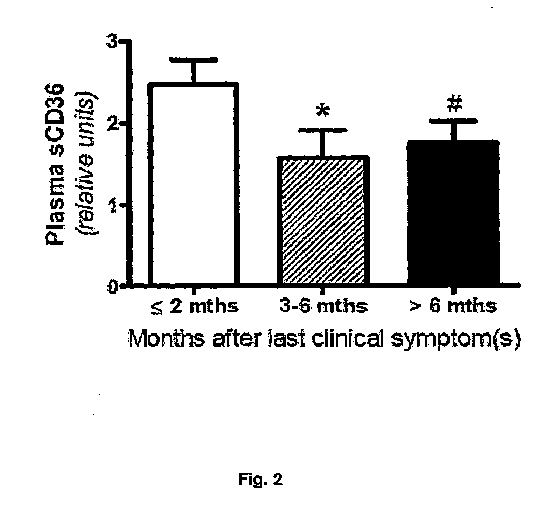

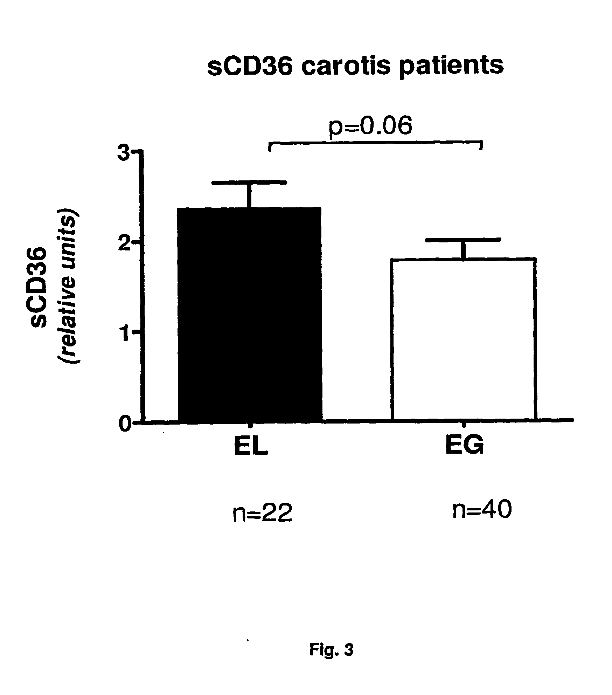

In contrast to CRP and neopterin, plasma levels of sCD36 were significantly higher in those who had symptoms related to their carotid stenosis within the last two months compared with the other patients (2.17 [0.58-4.74] versus 1.27[0-6.03], p=0.019, data are given in relative units as medians and ranges). Moreover, when patients were divided into three groups according to their latest clinical symptoms (i.e., symptoms within the last 2 months [n=16], symptoms within the last 3-6 months [n=15], or asymptomatic plaques, i.e., no symptoms or symptoms more than 6 months ago [n=31]), the former group had significantly raised plasma levels of sCD36 as compared with the other two groups (Figure 2). Within the patient group as a whole, plasma levels of sCD36 were significantly correlated with total leukocyte counts (r=0.43, p<0.02) and fibrinogen (r=-0.40. p<0.02). However, after adjustment for these parameters, sCD36 was still a significant predictor of clinical symptoms from the plaques within the last 2 months (p=0.049, Cl: 1.0-2.7). We have previously reported raised plasma levels of sCD36 in type 2 diabetes and in obese individuals, but in the present study we found no relationship between sCD36 and either BMI, the occurrence of diabetes mellitus or glycemic control (i.e., HbA1c) (data not shown).

We have previously demonstrated that CD36 exists in a soluble form in plasma, with elevated levels in patients with type 2 diabetes and in patients with the polycystic ovary syndrome in relation to risk factors for atherosclerosis such as insulin resistance and glycemic control (Handberg A et al. Circulation. 2006;114:1169-1176; Glintborg D et al. Diabetes Care. Published online ahead of print November 13, 2007. In the present study, we extended these findings by showing a more direct relationship between plasma levels of sCD36 and the nature of the atherosclerotic lesion, independent of BMI and the occurrence of diabetes. Thus, while there were no differences in traditional risk markers for cardiovascular disease or established markers of systemic inflammation and platelet activation between those with symptomatic disease in carotid arteries within the preceding 2 months and the other patients with carotid atherosclerosis, plasma levels of sCD36 were significantly raised in the former group of patients. Without being bound by theory one possible cellular source of sCD36 in these patients, based on our previous observation of increased CD36 mRNA levels in symptomatic carotid plaques combined with our present data showing strong immunostaining of CD36 in macrophages within symptomatic as compared asymptomatic plaques, may suggest that the raised plasma levels of sCD36 could reflect increased release from the symptomatic and unstable lesion.

### Example 4

### Plasma CD36 and plaque morphology

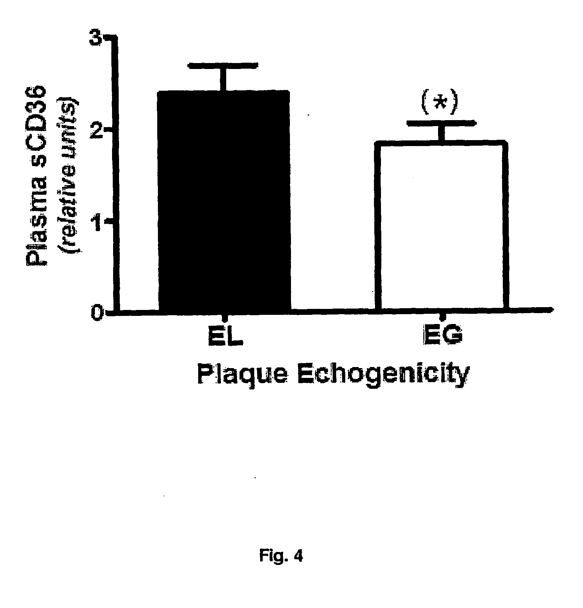

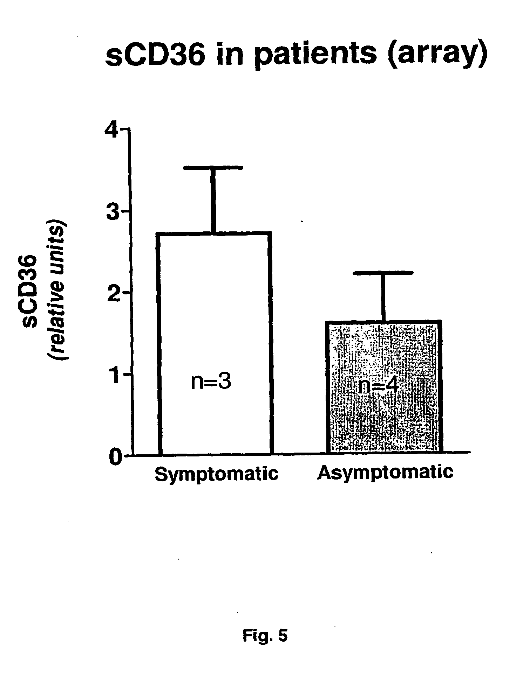

Ultrasound plaque appearance or echogenicity can in principle be classified into plaques with low-level echoes, with a thin often incomplete shell on the luminal surface (echolucent plaque), and plaques with medium and high level echoes often reflecting a higher content of dense fibrous tissue and calcification (echogenic/heterogeneous plaques). Data on plaque morphology were available in 59 of the patients, and as can be seen in Figure 4, patients with echolucent plaques (n=20) tended to have higher sCD36 levels compared with those with echogenic/heterogeneous plaques (n=39, p= 0.09).

### Example 5

### CD36 expression within the carotid lesion

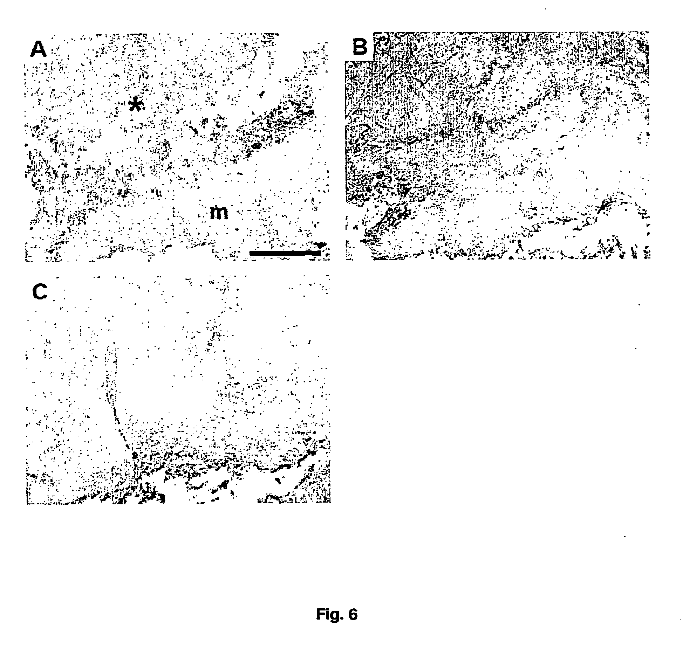

The cellular source of plasma levels of sCD36 in patients with symptomatic carotid lesions is not clear, but we have recently identified CD36 as one of 87 genes that was markedly up-regulated in symptomatic carotid plaques, suggesting that plaque in itself could contribute to the raised plasma levels of sCD36 in these patients. To further elucidate these issues, immunohistochemical analysis was performed on carotid plaques from 2 patients with symptomatic disease (<2 months) and 2 patients with asymptomatic disease (Figure 6). Staining of serial sections of these atherosclerotic lesions showed anti-CD36 immunostaining in plaques from symptomatic patients that were localized to the lipid-rich core of the plaque (Figure 6A) with strong immunostaining against calprotectin-positive macrophages (Figure 6B). Different intensity of CD36 immunoreactivity was seen in the core region with regions with very weak or no anti-CD36 immunostaining, as in the acellular center of the core, while other regions displayed more intense immunostaining. As demonstrated in Figure 6, regions of the plaque adjacent to the media displayed the strongest CD36 immunoreactivity. However, no anti-CD36 immunostaining was seen outside the core. CD36 immunostaining was also seen in lipid-rich calprotectin-positive regions in lesions from patients with asymptomatic disease, but in these patients, the atherosclerotic plaques were less advanced and the region with CD36-positive immunostaining was smaller than in lesions from patients with symptomatic disease.

CD36 is believed to play a critical role in the initiation and progression of atherosclerotic lesions through its ability to bind and internalize modified LDL trapped in the arterial wall, facilitating the formation of lipid-engorged macrophage foam cells (Collot-Teixeira S et al. Cardiovasc Res. 2007;75:468-477; Tuomisto TT et al. Atherosclerosis. 2005;180:283-291. Indeed, previous studies have reported accelerated CD36 expression in parallel with the progression of atherosclerosis 6, especially located to foamed, large-sized macrophages (Nakata Aet al. Arterioscler Thromb Vasc Biol. 1999;19:1333-1339. Our finding in the present study of strong immunostaining of CD36 in symptomatic as compared with asymptomatic plaques, primarily located to lipid-loaded macrophages in the fatty core of the atherosclerotic plaque, further support a relationship between enhanced CD36 and advanced atherosclerosis. Furthermore, plasma levels of sCD36 were markedly elevated in those with symptoms related to their carotid stenosis within the last two months as compared with other patients. The mechanisms for release of CD36 in its soluble form are at present unclear, but it has been suggested that plasma levels of sCD36 could serve as a biomarker of conditions with altered CD36 expression such as elevated levels of modified lipoproteins and low-grade inflammation (Tuomisto TT et al. Atherosclerosis. 2005;180:283-291).

Moreover, foam cell apoptosis has been linked to plaque destabilization and thrombus formation with subsequent development of acute ischemic events (Littlewood TD and Bennett. Curr OpinLipidol. 2003;14:469-475), and it may be speculated that apoptosis of lipid-loaded macrophages may lead to enhanced release of CD36. It is therefore tempting to hypothesize that the increased plasma levels of sCD36 in patients with recent symptomatic carotid plaques, with a time-dependent relationship with the acute symptoms, at least partly reflects intensified release of sCD36 during plaque destabilization.

CD36 is expressed not only on monocytes and macrophages, but also on endothelial cells Adachi H and Tsujimoto M. Prog Lipid Res. 2006;45:379-404 and platelets (Ikeda H. Hokkaido Igaku Zasshi. 1999;74:99- 104. Recently, Podrez and co-workers proposed that platelet-related CD36 could be a sensor of oxidative stress and a modulator of platelet activation, promoting thrombosis under hyperlipidemic conditions (Podrez EA et al. Nat Med. 2007;13:1086-1095) such as during interaction between platelets and an unstable atherosclerotic lesion. In contrast to sCD36, we found no relationship between plasma levels of CRP, neopterin, and beta-TG, which are reliable markers of systemic inflammation and monocyte/macrophage and platelet activation, respectively. It is possible that the ability of sCD36 to reflect plaque instability and symptomatic disease may reflect its capacity to mirror several pathogenic processes involved in plaque destabilization, such as activation of platelets and monocytes/macrophages, as well as pathogenic events within the lesion such as foam cell apoptosis and interactions between platelets and the atherosclerotic plaque.

We propose that sCD36 in plasma is a marker of plaque instability and symptomatic carotid atherosclerosis, possibly at least partly as a result of CD36 release into the circulation from the foam cell infiltrated layer overlaying the fatty core, and that this release is intensified during plaque rupture. The results of the present study suggest sCD36 to predict forthcoming cardiovascular events.

### References

Anderson GB, Ashforth R, Steinke DE, Ferdinandy R, Findlay JM. CT Angiography for the detection and characterization of carotid artery bifurcation disease, Strode. 2000; 31:2168-2174
Grant EG, Benson CB, Moneta GL, Alexandrov AV, Baker JD, Bluth El, Carroll BA, Eliasziw M, Gocke J, Hertzberg BS, Katarick S, Needleman L, Pellerito J, Polak JF, Rholl KS, Wooster DL, Zierler E. Carotid artery stenosis: gray-scale and doppler US diagnosis-Society of Radiologists in Ultrasound Consensus Conference, Radiology. 2003;229:340-346
European Carotid Plaque Study G, Carotid artery plaque composition-relationship to clinical presentation and ultrasound B-mode imaging, Eur J Vasc Endovasc Surg. 1995;10: 23-30
Mathiesen EB, Bonaa KH, Joakimsen O. Echolucent plaques are associated with high risk of ischemic cerebrovascular events in carotid stenosis: the Tromsø study, Circulation. 2001;103: 2171-2175
Handberg A, Levin K, Højlund K, Beck-Nielsen H. Identification of the oxidized low-density lipoprotein scavenger receptor CD36 in plasma: A novel marker of insulin resistance. Circulation. 2006;114:1169-1176
Glintborg D, Højlund K, Andersen M, Henriksen JE, Beck-Nielsen H, Handberg A. Soluble CD36 and risk markers of insulin resistance and atherosclerosis are elevated in polycystic ovary syndrome and significantly reduced during pioglitazone treatment. Diabetes Care. Published online ahead of print November 13,2007.
Collot-Teixeira S, Martin J, McDermott-Roe C, Poston R, McGregor JL. CD36 and macrophages in atherosclerosis. Cardiovasc Res. 2007;75:468-477
Tuomisto TT, Riekkinen MS, Viita H, Levonen AL, Yla-Herttuala S. Analysis of gene and protein expression during monocyte-macrophage differentiation and cholesterol loading-cDNA and protein array study. Atherosclerosis. 2005; 180:283-291.
Nakata A, Nakagawa Y, Nishida M, Nozaki S, Miyagawa J, Nakagawa T, Tamura R, Matsumoto K, Kameda-Takemura K, Yamashita S, Matsuzawa Y. CD36, a novel receptor for oxidized low-density lipoproteins, is highly expressed on lipidladen macrophages in human atherosclerotic aorta. Arterioscler Thromb Vasc Biol. 1999;19:1333-1339.
Littlewood TD, Bennett MR. Apoptotic cell death in atherosclerosis. Curr Opin Lipidol. 2003;14:469-475.
Adachi H, Tsujimoto M. Endothelial scavenger receptors. Prog Lipid Res. 2006; 45:379-404.
Ikeda H. Platelet membrane protein CD36. Hokkaido Igaku Zasshi. 1999;74:99-104.
Podrez EA, Byzova TV, Febbraio M, Salomon RG, Ma Y, Valiyaveettil M, Poliakov E, Sun M, Finton PJ, Curtis BR, Chen J, Zhang R, Silverstein RL, Hazen SL. Platelet CD36 links hyperlipidemia, oxidant stress and a prothrombotic phenotype. Nat Med. 2007;13:1086-1095

## Claims

1. A method for diagnosing atherosclerotic plaques as instable and/or diagnosing stenosis caused by instable atherosclerotic plaques in an individual, said method comprising in a blood sample from said individual
i) determining the concentration of a soluble CD36 polypeptide,
ii) correlating said concentration determined in i) to a standard level, and
iii) based on said correlation according to ii) diagnosing said atherosclerotic plaques as instable and/or diagnosing stenosis caused by instable atherosclerotic plaques.

2. The method according to claim 1, wherein said sample is a cell-free sample.

3. The method according to any of the preceding claims, wherein said sample is a plasma sample.

4. The method according to any of the preceding claims, wherein an increase of the CD36 concentration of at least 1.15 of the standard level is an indication of an atherosclerotic plaque in progression.

5. The method according to any of the preceding claims, wherein said concentration of said CD36 polypeptide or part thereof is determined in
i) an amino acid sequence with SEQ ID NO: 4,
ii) an amino acid sequence having at least 90 % sequence identity with a sequence of (i).

6. The method according to any of the preceding claims, wherein the CD36 polypeptide concentration is determined by
i) providing an anti-CD36 antibody,
ii) exposing the sample to the anti-CD36 antibody
iii) optionally, exposing said CD36-antibody complex to at least a further antibody directed against said CD36-antibody complex, and
iv) detecting and quantifying the amount of said antibody

7. The method according to any of the preceding claims, wherein the CD36 polypeptide is determined by,
i) providing an anti-CD36 antibody,
ii) exposing the sample to be investigated to the anti-CD36 antibody bound to a solid phase,
iii) optionally exposing the CD36-antibody complex to a second anti-CD36 antibody, and
iv) detecting and quantifying the amount of the antibody which binds to CD36.

8. The method according to any of claims 5 to 6, wherein said immunological method is a solid phase ELISA enzyme immunoassay wherein in step (iii) the sample is contacted with said antibody bound to a solid phase.

9. The method of claim 7, wherein the solid phase is a microtiter plate.

10. The method of any one of claims 5 to 8, wherein said anti-CD36 antibody is selected from the group consisting of monoclonal and polyclonal CD36 specific antibodies.

11. The method of claim 9, wherein said anti-CD36 antibody is selected from the group of antibodies consisting of sc5522 (CD36 (N-15), goat IgG (epitope N-terminus (h)), sc9154 (CD36 (H-300), rabbit IgG (epitope 1-300 (h)), and sc7309 (CD36 (SMf), mouse IgM).

12. Use of a kit for the method as defined in claim 1, comprising at least one antibody directed against CD36 polypeptide or part thereof.

## Patentansprüche

1. Verfahren zum Diagnostizieren von arteriosklerotischen Plaques als instabil und/oder Diagnostizieren von durch instabile arteriosklerötische Plaques verursachter Stenose bei einem Individuum, wobei das Verfahren umfasst, in einer Blutprobe des Individuum
i) die Konzentration eines löslichem CD36-Polypeptids zu bestimmen,
ii) die in i) bestimmte Konzentration mit einem Standardspiegel zur korrelieren und
iii) beruhend auf der Korrelation gemäß ii) die arteriosklerotischen Plaques als instabil zu diagnostizieren und/oder eine durch instabile arteriosklerotische Plaques verursachte Stenose zu diagnostizieren.

2. Verfahren gemäß Anspruch 1, wobei es sich bei der Probe um eine zellfreie Probe handelt,

3. Verfahren gemäß einem der vorhergehenden Ansprüche, wobei es sich bei der Probe um eine Plasmaprobe handelt.

4. Verfahren gemäß einem der vorhergehenden Ansprüche, wobei eine Erhöhung der CD36-Konzentration von mindestens 1,15 des Standardspiegels ein Anzeichen für einen fortschreitenden arteriosklerotischen Plaques ist.

5. Verfahren gemäß einem der vorhergehenden Ansprüche, wobei die Konzentration des CD36-Polypeptids oder Teils davon in
i) einer Aminosäuresequenz mit der SEQ ID NO: 4,
ii) einer Aminosäuresequenz die mindestens 90% Sequenzidentität mit einer Sequenz nach (1) aufweist,
bestimmt wird.

6. Verfahren gemäß einem der vorhergehenden Ansprüche, wobei die CD36-Polypeptid-Konzentration bestimmt wird durch
i) Bereitstellen eines anti-CD36-Antikörpers,
ii) Behandelt der Probe mit dem anti-CD36-Antikörper,
iii) gegebenenfalls Behandeln des CD36-Antikörper-Komplexes mit mindestens einem weiteren gegen den CD36-Antikörper-Komplex gerichteten Antikörper und
iv) Nachweisen und Quantifizieren der Menge des Antikörpers.

7. Verfahren gemäß einem der vorhergehenden Ansprüche, wobei das CD36-Polypeptid bestimmt wird durch
i) Bereitstellen eines anti-CD36-Antikörpers,
ii) Behandeln der zu untersuchenden Probe mit dem an eine feste Phase gebundenen anti-CD36-Antikörper,
iii) gegebenenfalls Behandeln des CD36-Antikörper-Komplexes mit einem zweiten anti-CD36-Antikörper und
iv) Nachweisen und Quantifizieren der Menge des Antikörpers, der an CD36 bindet.

8. Verfahren gemäß einem der Ansprüche 5 bis 6, wobei es sich bei dem immunologischen Verfahren um einen Festphasen-ELISA-Enzymimmunoassay handelt, wobei in Schritt (iii) die Probe mit dem an eine feste Phase gebundenen Antikörper in Kontakt gebracht wird.

9. Verfahren nach Anspruch 7, wobei es sich bei der festen Phase um eine Mikrotiterplatte handelt.

10. Verfahren nach einem der Ansprüche 5 bis 8, wobei der anti-CD36-Antikörper aus der Gruppe ausgewählt ist, bestehend aus monoklonalen und polyklonalen CD36-spezifischen Antikörpern.

11. Verfahren nach Anspruch 9, wobei der anti-CD36-Antikörper aus der Antikörpergruppe ausgewählt ist, bestehend aus sc5522 (CD36 (N-15), Ziegen-IgG (Epitop N-Terminus (h)), sc9154 (CD36 (H-300), Kaninchen-IgG (Epitop 1-300 (h)) und sc7309 (CD36 (SMf), Maus-IgM).

12. Verwendung eines Kits für das wie in Anspruch 1 definierte Verfahren, umfassend mindestens einen gegen ein CD36-Polypeptid oder einen Teil davon gerichteten Antikörper.

## Revendications

1. Procédé de diagnostic de plaques athéroscléreuses comme instables et/ou de diagnostic d'une sténose provoquée par des plaques athéroscléreuses instables chez un individu, ledit procédé comprenant dans un échantillon de sang provenant dudit individu
i) la détermination de la concentration d'un polypeptide CD36 soluble,
ii) la corrélation de ladite concentration déterminée en i) avec une concentration étalon, et
iii) en se basant sur ladite corrélation selon ii), le diagnostic desdites plaques athéroscléreuses comme instables et/ou le diagnostic d'une sténose provoquée par des plaques athéroscléreuses instables.

2. Procédé selon la revendication 1, dans lequel ledit échantillon est un échantillon acellulaire.

3. Procédé selon l'une quelconque des revendications précédentes, dans lequel ledit échantillon est un échantillon de plasma.

4. Procédé selon l'une quelconque des revendications précédentes, dans lequel une augmentation de la concentration de CD36 d'au moins 1,15 de la concentration étalon est une indication d'une plaque athéroscléreuse en progression.

5. Procédé selon l'une quelconque des revendications précédentes, dans lequel ladite concentration dudit polypeptide CD36 ou d'une partie de celui-ci est déterminée dans
i) une séquence d'acides aminés avec SEQ ID NO : 4,
ii) une séquence d'acides aminés présentant au moins 90 % d'identité de séquence avec une séquence de (i).

6. Procédé selon l'une quelconque des revendications précédentes, dans lequel la concentration dudit polypeptide CD36 est déterminée par
i) la fourniture d'un anticorps anti-CD36,
ii) l'exposition de l'échantillon à l'anticorps anti-CD36,
iii) éventuellement, l'exposition dudit complexe CD36-anticorps à au moins une autre anticorps dirigé contre ledit complexe CD36-anticorps, et
iv) la détection et la quantification de la quantité dudit anticorps.

7. Procédé selon l'une quelconque des revendications précédentes, dans lequel le polypeptide CD36 est déterminé par
i) la fourniture d'un anticorps anti-CD36,
ii) l'exposition de l'échantillon à étudier à l'anticorps anti-CD36 lié à une phase solide,
iii) éventuellement l'exposition du complexe CD36-anticorps à un second anticorps anti-CD36, et
iv) la détection et la quantification de la quantité de l'anticorps qui se lie au CD36.

8. Procédé selon l'une quelconque des revendications 5 ou 6, dans lequel ledit procédé immunologique est un test immuno-enzymatique LISA sur phase solive dans lequel dans l'étape (iii), l'échantillon est mis en contact avec ledit anticorps lié à une phase solide.

9. Procédé selon la revendication 7, dans lequel ladite phase solide est une plaque de microtitrage.

10. Procédé selon l'une quelconque des revendications 5 à 8, dans lequel ledit anticorps anti-CD36 est choisi dans le groupe constitué d'anticorps monoclonaux et polyclonaux spécifiques du CD36.

11. Procédé selon la revendication 9, dans lequel redit anticorps anti-CD36 est choisi dans le groupe d'anticorps constitué de sc5522 (CD36 (N-15), IgG de chèvre (épitope N-terminal (h)), sc9154 (CD36 (H-300), IgG de lapin (épitoge 1-300 (h)), et sc7309 (CD36 (SMf), IgM de souris).

12. Utilisation d'un kit pour le procédé tel que défini dans la revendication 1, comprenant au moins un anticorps dirigé contre le polypeptide CD36 ou une partie de celui-ci.
